(19) Europäisches Patentamt
European Patent Office
Office européen des brevets

(11) **EP 1 648 493 B1**

(12) **EUROPEAN PATENT SPECIFICATION**

(45) Date of publication and mention of the grant of the patent:
**09.12.2009 Bulletin 2009/50**

(51) Int Cl.:
*A61K 38/17* (2006.01)    *A61K 38/18* (2006.01)
*A61K 38/19* (2006.01)    *A61K 38/21* (2006.01)
*A61K 45/06* (2006.01)    *A61K 35/00* (2006.01)

(21) Application number: **04777630.7**

(22) Date of filing: **07.07.2004**

(86) International application number:
**PCT/US2004/021641**

(87) International publication number:
**WO 2005/007193 (27.01.2005 Gazette 2005/04)**

(54) **INHIBITION OF TUMOR ANGIOGENESIS BY COMBINATION OF THROMBOSPONDIN-1 AND INHIBITORS OF VASCULAR ENDOTHELIAL GROWTH FACTOR**

HEMMUNG DER TUMOR-ANGIOGENESE DURCH EINE KOMBINATION VON THROMBOSPONDIN-1 UND HEMMERN DES VASKULÄREN ENDOTHEL-WACHSTUMSFAKTORS

INHIBITION DE L'ANGIOGENESE TUMORALE PAR COMBINAISON DE LA THROMBOSPONDINE-1 ET DES INHIBITEURS DU FACTEUR DE CROISSANCE ENDOTHELIALE VASCULAIRE

(84) Designated Contracting States:
**AT BE BG CH CY CZ DE DK EE ES FI FR GB GR HU IE IT LI LU MC NL PL PT RO SE SI SK TR**

(30) Priority: **07.07.2003 US 484676 P**

(43) Date of publication of application:
**26.04.2006 Bulletin 2006/17**

(73) Proprietor: **Van Andel Research Institute Grand Rapids, MI 49503 (US)**

(72) Inventor: **ZHANG, Yu-Wen Grand Rapids, MI 49503 (US)**

(74) Representative: **van Westenbrugge, Andries Nederlandsch Octrooibureau Postbus 29720 2502 LS Den Haag (NL)**

(56) References cited:
WO-A-01/66144    WO-A-02/18379
WO-A-02/18380    WO-A-99/43311
WO-A-02/076496    WO-A-03/018748
US-A- 5 639 725    US-A- 5 707 624
US-A- 5 997 868    US-B1- 6 576 632

• ALLEGRINI G ET AL: "THE ANGIOGENESIS INHIBITOR THROMBOSPONDIN-1 PLUS IRINOTECAN SIGNIFICANTLY INHIBIT TUMOR GROWTH IN HUMAN COLON TUMOR BEARING NUDE MICE" PROCEEDINGS OF THE 91ST ANNUAL MEETING OF THE AMERICAN ASSOCIATION FOR CANCER RESEARCH. SAN FRANCISCO, CA, APRIL 1 - 5, 2000, PROCEEDINGS OF THE ANNUAL MEETING OF THE AMERICAN ASSOCIATION FOR CANCER RESEARCH, PHILADELPHIA, PA : AACR, US, vol. 41, March 2000 (2000-03), page 813, XP001019254

• F.Y.F.L DE VOS: "A phase I dose escalating study of the angiogenesis inhibitor thrombospondin-1 mimetic (abt-510) in patients with advanced cancer" EUROPEAN JOURNAL OF CANCER, PERGAMON PRESS, OXFORD, GB, vol. 38, November 2002 (2002-11), pages S78-S79, XP004403691 ISSN: 0959-8049

• ARMSTRONG L C ET AL: "Thrombospondins 1 and 2 function as inhibitors of angiogenesis" MATRIX BIOLOGY, ELSEVIER, vol. 22, no. 1, March 2003 (2003-03), pages 63-71, XP002982095 ISSN: 0945-053X

• GUO N-H ET AL: "ANTIPROLIFERATIVE AND ANTITUMOR ACTIVITIES OF D-REVERSE PEPTIDES DERIVED FROM THE SECOND TYPE-1 REPEAT OF THROMBOSPONDIN-1" JOURNAL OF PEPTIDE RESEARCH, MUNKSGAARD INTERNATIONAL PUBLISHERS, COPENHAGEN, DK, vol. 50, no. 3, September 1997 (1997-09), pages 210-221, XP000696384 ISSN: 1397-002X

- STREIT MICHAEL ET AL: "Overexpression of thrombospondin-1 decreases angiogenesis and inhibits the growth of human cutaneous squamous cell carcinomas" AMERICAN JOURNAL OF PATHOLOGY, vol. 155, no. 2, August 1999 (1999-08), pages 441-452, XP001204804 ISSN: 0002-9440
- REIHER FRANK K ET AL: "Inhibition of tumor growth by systemic treatment with thrombospondin-1 peptide mimetics" INTERNATIONAL JOURNAL OF CANCER, vol. 98, no. 5, 10 April 2002 (2002-04-10), pages 682-689, XP001204803 ISSN: 0020-7136
- FEIGE J J: "The thrombospondins: Multimodular proteins with angiogenesis inhibiting effects" PATHOLOGIE BIOLOGIE, vol. 47, no. 4, April 1999 (1999-04), pages 339-344, XP009042623 ISSN: 0369-8114
- LAWLER JACK: "Thrombospondin-1 as an endogenous inhibitor of angiogenesis and tumor growth" JOURNAL OF CELLULAR AND MOLECULAR MEDICINE, vol. 6, no. 1, January 2002 (2002-01), pages 1-12, XP009042620 ISSN: 1582-1838
- HENKIN JACK ET AL: "Tumor inhibition by anti-angiogenic TSP-1 mimetic peptides" PROCEEDINGS OF THE AMERICAN ASSOCIATION FOR CANCER RESEARCH ANNUAL MEETING, vol. 43, March 2002 (2002-03), page 180, XP001204805 & 93RD ANNUAL MEETING OF THE AMERICAN ASSOCIATION FOR CANCER RESEARCH; SAN FRANCISCO, CALIFORNIA, USA; APRIL 06-10, 2002 ISSN: 0197-016X
- VAILHÉ BRUNO ET AL: "Thrombospondins as anti-angiogenic therapeutic agents." CURRENT PHARMACEUTICAL DESIGN. 2003, vol. 9, no. 7, 2003, pages 583-588, XP1204808 ISSN: 1381-6128
- IRUELA-ARISPE M L ET AL: "INHIBITION OF ANGIOGENESIS BY THROMBOSPONDIN-1 IS MEDIATED BY 2 INDEPENDENT REGIONS WITHIN THE TYPE 1 REPEATS" CIRCULATION, AMERICAN HEART ASSOCIATION, DALLAS, TX, US, vol. 100, no. 13, 28 September 1999 (1999-09-28), pages 1423-1431, XP000923386 ISSN: 0009-7322
- DATABASE WPI Section Ch, Week 200347 Derwent Publications Ltd., London, GB; Class B04, AN 2003-496683 XP002314426 & JP 2003 012541 A (FUJI PHARM IND CO LTD) 15 January 2003 (2003-01-15)
- DATABASE WPI Section Ch, Week 200364 Derwent Publications Ltd., London, GB; Class B03, AN 2003-674472 XP002314427 & JP 2003 183249 A (RIKAGAKU KENKYUSHO) 3 July 2003 (2003-07-03)
- ZHANG YU-WEN ET AL: "Hepatocyte growth factor/scatter factor mediates angiogenesis through positive VEGF and negative thrombospondin 1 regulation." PROCEEDINGS OF THE NATIONAL ACADEMY OF SCIENCES OF THE UNITED STATES OF AMERICA, vol. 100, no. 22, 28 October 2003 (2003-10-28), pages 12718-12723, XP001204754 ISSN: 0027-8424

Remarks:
The file contains technical information submitted after the application was filed and not included in this specification

**Description**

## BACKGROUND OF THE INVENTION

Field of the Invention

The present invention, in the field of cell and molecular biology and medicine is directed to methods for inhibiting tumor angiogenesis, and thereby, suppressing or preventing tumor growth or metastasis by the combination of anti-angiogenic factors such as Thormobospondin-1 (TSP-1) and inhibitors of Vascular endothelial growth factor (VEGF) .

## Description of the Background Art

Hepatocyte growth factor/scatter factor (HGF/SF) and its tyrosine kinase receptor, Met, have been associated with most types of the major human cancers and expression is often correlated with poor prognosis and metastasis (1, 2). Constitutively active mutations in Met, either sporadic or inherited, have been found in human cancers, providing strong genetic evidence for the role of Met in human malignancies (1). Multiple biological activities of HGF/SF-Met signaling account for its role in cancer, among which, most critical, are cell proliferation, tumor cell invasion and angiogenesis (1). Angiogenesis is an essential component for tumor development (3) and both angiogenic and anti-angiogenic factors have been characterized (4). Vascular endothelial growth factor (VEGF) is a potent agonist of angiogenesis and has been shown to activate both endothelial cell proliferation and migration (5). VEGF acts as a potent endothelial cell mitogen and key regulator of both physiologic and pathologic angiogenesis.

By contrast, thrombospondin-1 (TSP-1) is an angiogenesis antagonist and suppresses angiogenesis by inhibiting endothelial cell proliferation and inducing apoptosis (6, 7).

Furthermore, the use of TSP-1 for inhibition of angiogenesis is disclosed in each of WO 01/66144, Allegrini et al. (2000, Proc. Ann. Meeting of the American Association for Cancer Res. 41: 813), de Vos et al. (2002, Eur. J. Cancer 38: 878-879), Armstrong et al. (2003, Matrix Biol. 22: 63-71), Guo et al. (1997, J. Peptide Res. 50: 210-221), Streit et al. (1999, American J. Pathol. 155: 441-452), Reiher et al. (2002, Int. J. Cancer 98: 682-689) Feige (Pathologie Biologie 47: 339-344), Lawler (2002, J. Cell. Mol. Medicin 6: 1582-1838), Henkin et al. (2002, Proc. Ann. Meeting of the American Association for Cancer Res. 43: 180), Vailé et al. (2003, Curr. Pharm. Design 9: 583-588) and Iruela-Arispe et al. (1999, Circulation 100: 1423-1431). Previously, it has been shown that TSP-1 expression is positively regulated by the p53 tumor suppressor protein (8). Many cells express TSP-1 and low levels of TSP-1 expression has been associated with increased cancer recurrence rates and decreased overall survival in several human cancers (6), suggesting that TSP-1 has an important inhibitory role in tumor development. Overexpression of TSP-1 in human skin carcinoma cells has been shown to suppress tumor progression through inhibition of angiogenesis (9). Thus, VEGF and TSP-1 can contribute to angiogenic switching where angiogenesis depends on which of the angiogenic effectors becomes dominant (4).

HGF/SF induces angiogenesis, the ligand stimulates endothelial cells to proliferate and migrate *in vitro*, induces blood vessel formation *in vivo* (10-12) and induces the expression of VEGF in human cancer cells (13, 14). Here the present inventors show that HGF/SF-Met signaling operates as a true angiogenic switch, turning on VEGF and turning off TSP-1 expression.

Hanahan and Folkman have emphasized the importance of angiogenesis for tumor development (4) and much effort has been directed to blocking this tumor growth dependent organogenesis. Many angiogenesis inhibitors have been characterized and some are in clinical trials (21). TSP-1 is a candidate with potential clinical utility, while a neutralizing monoclonal antibody ("mAb") to VEGF (Avastin®), which inhibits tumor angiogenesis, looks promising in clinical trials (22; Wall Street Journal).

MAPK Pathway

The MAPK pathways are found in, and highly conserved among, all eukaryotes. These pathways play an integral role in the transduction of various extracellular signals into the nucleus. The best-characterized mammalian pathway, designated **Raf-MEK1/2-ERK1/2,** includes the MAPK enzymes also known as ERK1 and ERK2, which are phosphorylated and activated by the dual-specificity kinases that have been termed "MAPK/ERK kinases" (abbreviated variously as MAPKK1 and MAPKK2 or, as will be used herein, MEK1 and MEK2). The MEK enzymes are in turn phosphorylated and activated by the Raf kinases (Lewis, TS. et al., Adv Canc Res, 74:49-139 (1998)).

The MAPK pathway is involved in the regulation of cell growth, survival, and differentiation (Lewis *et al., supra*). Furthermore, activated MAPK and/or elevated level of MAPK expression have been detected in a variety of human tumors (Hoshino, R. et al., Oncogene 18:813-822 (1999); Salh, B et al., Anticancer Res. 19:741-48 (1999); Sivaraman, VS et al., J. Clin. Invest. 99:1478-483 (1997); Mandell, JW et al., Am. J. Patrol. 153:1411-23 (1998); Licato, L.L. et al. Digestive Diseases and Sciences 43, 1454-1464 (1998)) and may be associated with invasive, metastatic and angiogenic activities of tumor cells. Thus, inappropriate activation of the MAPK pathway is an essential feature common to many types of tumors. For this reason, participants in this signaling pathway, such as MEK, are potential targets for cancer therapy.

However, it has generally been observed that inhibitors of signal transduction, including of the MAPK pathway, are cytostatic in nature, merely arresting the growth of tumor cells but not killing them, creating an expectation that non-traditional approaches would be required to develop such agents into clinical therapeutics.

The present invention is directed to improved methods of inhibiting tumor angiogenesis, and thereby, tumor growth and metastasis, and for treating a subject with either a Met-positive or a Met-negative human tumor.

Citation of the above documents is not intended as an admission that any of the foregoing is pertinent prior art. All statements as to the date or representation as to the contents of these documents is based on the information available to the applicant and does not constitute any admission as to the correctness of the dates or contents of these documents.

## SUMMARY OF THE INVENTION

HGF/SF can induce cell proliferation, invasion and angiogenesis, all of which are essential biological components for tumor malignancy. Targeting on either component may have effects on tumor progression. The mechanism underlying HGF/SF-induced tumor angiogenesis has not been fully explained. Angiogenesis is switched on or off by the balance of angiogenic and anti-angiogenic factors. HGF/SF induces the expression of VEGF, an angiogenic factor, in certain tumor cells.

The present inventors are the first to discover that, in addition to the induction of VEGF, HGF/SF down-regulates the expression of TSP-1, an anti-angiogenic factor in the very same tumor cells. In addition, in the normal human umbilical vein endothelial cells (HUVEC), HGF/SF also decreases the expression of TSP-1, while VEGF expression is undetectable.

According to the present invention, down-regulation of TSP-1 plays an important role in HGF/SF-mediated tumor development as overexpression of TSP-1 significantly inhibited tumor progression through suppression of angiogenesis.

Also shown herein, TSP-1 down-regulation by HGF/SF is prevented uniquely by inhibiting MAP kinase activation, while VEGF induction is suppressed by the inhibitors of several pathways, including MAP kinase, PI3 kinase and Stat3.

These results provide a further insight into the mechanism of how HGF/SF induces tumor angiogenesis, and the first evidence that the MAP kinase pathway plays a dual role in regulating angiogenic effectors and offer a strong molecular basis for using MAP kinase inhibitor in inhibiting tumor angiogenesis.

TSP-1, as well as biologically active TSP-1 peptides that possess the antiangiogenic activity of the intact protein, and TSP-1 mimics or mimetics, including peptidomimetics (referred to collectively as "TSP-1 agonists"), are useful antagonists to tumorigenesis and can be employed therapeutically, preferably in conjunction with (a) an inhibitor of VEGF, preferably VEGF-Trap or anti-VEGF mAb such as Avastin®, (b) an inhibitor of HGF, such as an anti-HGF mAb, or both a VEGF inhibitor and an HGF inhibitor..

A combination treatment with VEGF-Trap or anti-VEGF neutralizing antibody plus a therapeutic TSP-1 agonist synergizes to inhibit tumor angiogenesis and, therefore, tumor growth.

A combination of drugs that target TSP-1 and VEGF expression dependent signaling pathways are used as therapeutic agents. These combinations are particularly effective because the MAP kinase pathway plays a dual role in the negative regulation of TSP-1 expression and the up-regulation of VEGF expression by HGF/SF. Therefore, MAP kinase inhibitors are effective clinical tools to inhibit or prevent tumor angiogenesis.

Specifically, the present invention provides a method of inhibiting angiogenesis, preferably tumor angiogenesis, comprising providing to cells that undergo angiogenesis or participate in angiogenesis, or to a subject in need thereof, an effective amount or amounts of one of more of:

    (a) an anti-angiogenic factor or anti-angiogenic agonist; and
    (b) an inhibitor of angiogenic protein or pathway;
    wherein the factor or agonist of (a) and the inhibitor of (b)

        (i) inhibits endothelial cell proliferation,
        (ii) inhibits endothelial cell migration, and/or
        (iii) induces endothelial cell apoptosis

    thereby inhibiting the angiogenesis. The compound comprises the factor or agonist of (a) and the inhibitor of (b), above.

In the above method or composition, the anti-angiogenic factor or agonist is TSP-1 or an anti-angiogenically functional derivative thereof. The angiogenic protein of (b) that is being inhibited is VEGF.

Preferably, the inhibitor of (b) is a VEGF inhibitor that inhibits VEGF expression or action, or inhibits expression or action of VEGF receptors. Examples of such VEGF or VEGF-receptor inhibitors include an anti-VEGF antibody, an anti-VEGF receptor antibody, a decoy VEGF receptor, VEGF-Trap, a siRNA specific for VEGF, a siRNA specific for VEGF receptor, or a peptidomimetic inhibitor of VEGF receptor activation. Most preferred is an anti-VEGF mAb, preferably the mAb termed Avastin®.

In the above methods, the providing may be to a subject *in vivo*, which subject is susceptible to, or at risk of, tumor growth or metastasis, or in which subject the tumor growth or metastasis is ongoing.

Preferably the above method comprises providing effective amounts of (A) TSP-1 or a TSP-1 agonist or mimic, preferably TSP-1, in combination with (B) VEGF-Trap or, preferably, an anti-VEGF antibody, most preferably Avastin®.

Also included are pharmaceutical compositions comprising a composition as described above and, further, a pharmaceutically acceptable vehicle or excipient.

## BRIEF DESCRIPTION OF THE DRAWINGS

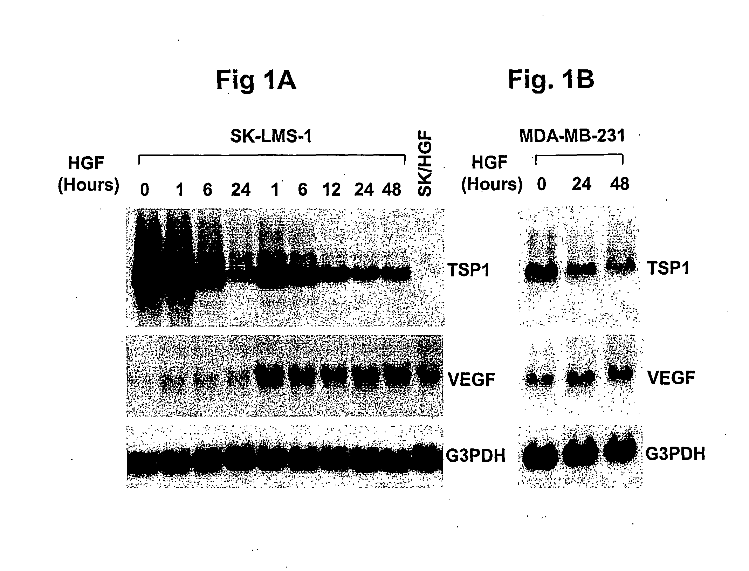

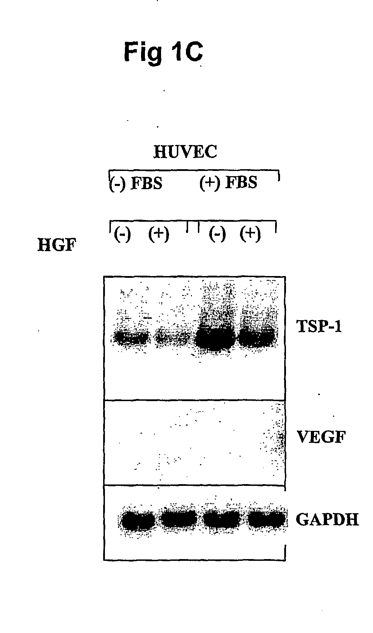

Fig. 1A-1C. HGF/SF up-regulates VEGF and down-regulates TSP-1 expression in SK-LMS-1 cells (Fig. 1A) and MDA-MB-231 cells (Fig. 1B) and HUVEC cells (Fig. 1C). Total RNAs were prepared from SK-LMS-1 cells, MDA-MB-231 cells or HUVEC with or without treatment of recombinant human HGF/SF (200 units/ml) at the indicated time points after stimulation. Total RNAs were also prepared from the SK/HGF cell line, a long-term culture derivative of SK-LMS-1 cells that is autocrine for human HGF/SF (15). Northern Blot was probed with $^{32}$P-radiolabeled TSP-1, VEGF or G3PDH cDNA fragment, respectively. For HUVEC, treatment of recombinant human HGF/SF (200 units/ml) was for 24 hours in the presence or absence of fetal bovine serum (FBS) and Northern Blot analyses were performed using $^{32}$P-radiolabelled probe for human TSP-1, VEGF or GAPDH, respectively. TSP-1 expression in HUVEC cells was decreased in response to HGF/SF treatment. However, VEGF expression was undetectable with or without HGF/SF treatment.

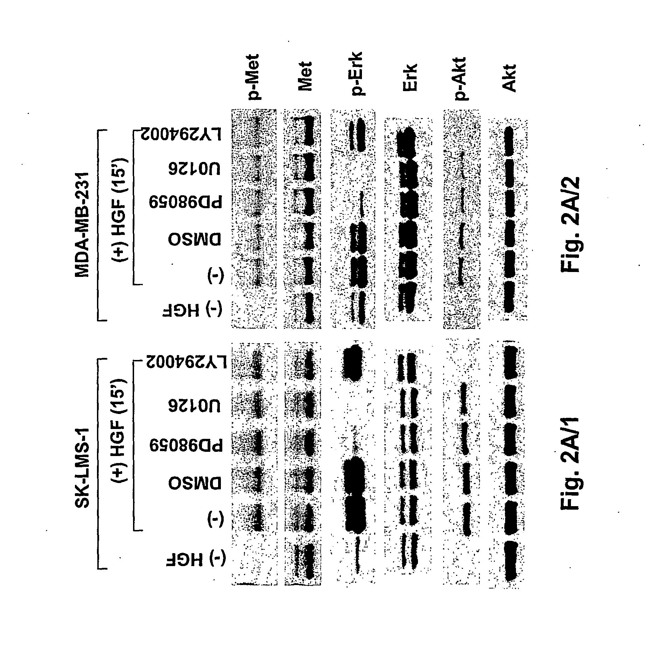

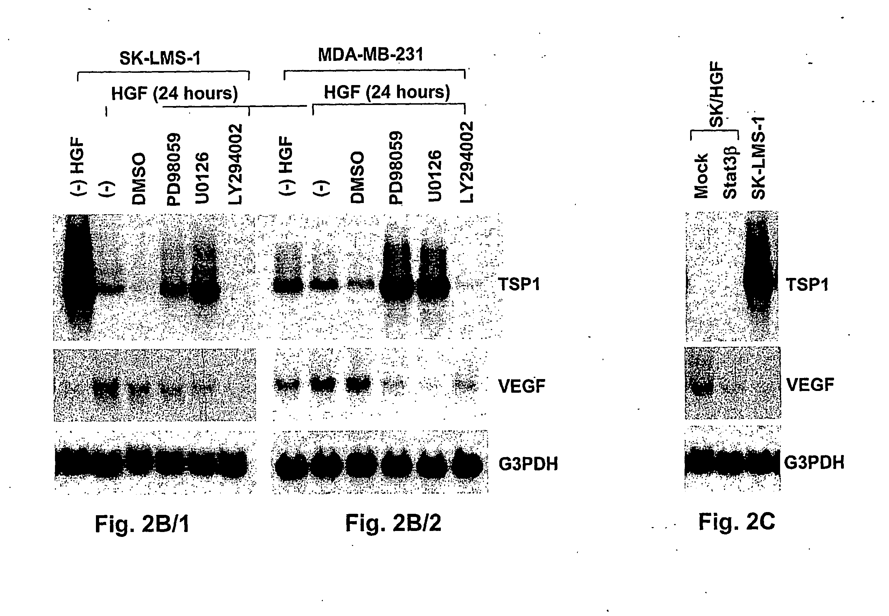

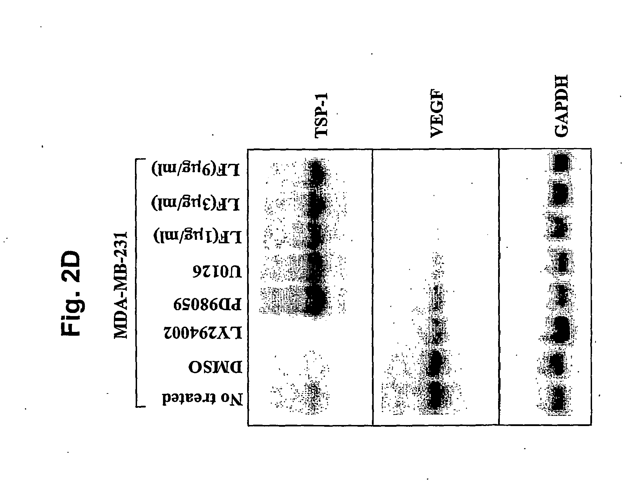

Fig. 2A-2D demonstrate how HGF/SF-Met signaling pathways regulate TSP-1 and VEGF expression. Fig. 2A: HGF/SF induces activation of MAP kinase and PI3 kinase pathways in SK-LMS-1 cells and MDA-MB-231 cells. Serum-starved cells were treated with or without DMSO (control), PD98059 (80 $\mu$M), U0126 (40 $\mu$M) or LY294002 (40 $\mu$M) for 1 hour, followed by HGF/SF stimulation for 15 minutes (A time point good for observing all the tyrosine phosphorylation statuses). Whole cell extracts were prepared and the state of Met phosphorylation was detected by immunoprecipitation with anti-human Met antibody, followed by Western blot with anti-Phosphotyrosine (and/or anti-human Met antibody). For detection of Erk and Akt, Western blots were probed with anti-phospho p44/42 MAPK, anti-p44/42 MAPK, anti-phospho Akt (Ser473) or anti-Akt antibodies, respectively. Fig. 2B: Negative regulation of TSP-1 expression occurs primarily through the MAP kinase pathway, while positive regulation of VEGF expression occurs through MAP kinase and PI3 kinase pathway. Total RNAs were prepared from cells with or without inhibitor treatment and/or HGF/SF treatment. Northern Blot analyses were performed as described in Fig. 1A- caption. Down-regulation of TSP-1 by HGF/SF was inhibited by the MAP kinase inhibitors, either PD98059 or U0126, but not affected by LY294002. Up-regulation of VEGF was inhibited by PD98059, U0126 as well as LY294002. Fig. 1C: VEGF but not TSP-1 expression was regulated by Stat3 signaling. Total RNAs were prepared from SK/HGF cells with or without overexpression of a dominant negative form of Stat3, Stat3β (17). Overexpression of Stat3β decreased VEGF expression but did not affect TSP-1 expression in SK/HGF cells. Fig. 2D: LF increases TSP-1 and decreases VEGF expression in MDA-MB-231 human breast cancer cells. Cells were treated with or without indicated inhibitors for 24 hours and then total RNAs were prepared. Northern Blot analyses were performed using $^{32}$P-radiolabelled probe for human TSP-1, VEGF or GAPDH, respectively. LF (at 1, 3 or 9 $\mu$g/ml) shows more dramatic effect on inhibiting VEGF expression, while displaying similar effect on inducing TSP-1 expression, compared to MAPK inhibitor PD98059 or U0126.

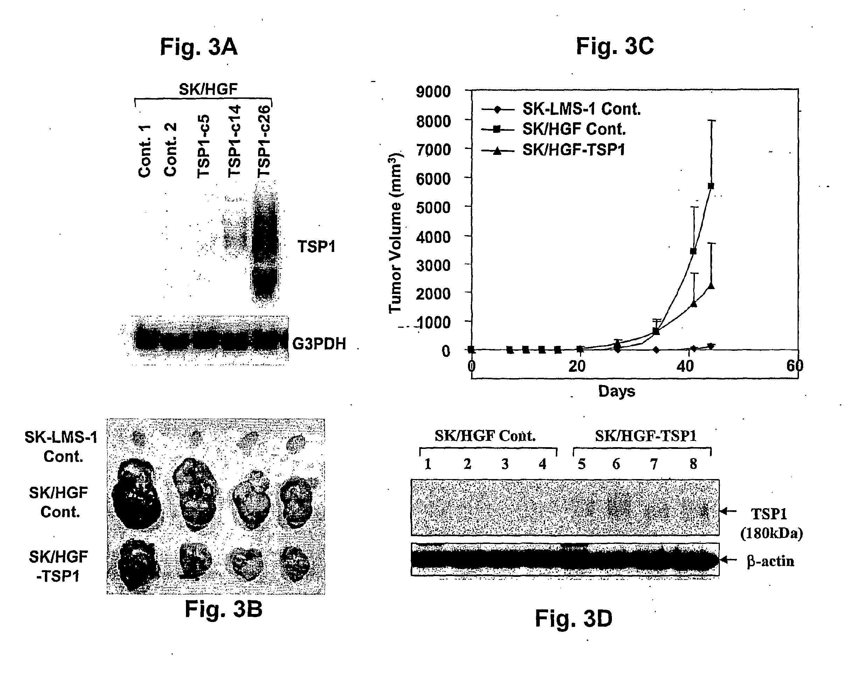

Fig. 3A-3D. TSP-1 inhibits HGF/SF-induced tumor growth *in vivo.* Fig. 3A: TSP-1 was ectopically expressed in SK/HGF cells, establishing the SK/HGF-TSP1 cell line. The expression of TSP-1 in SK/HGF-TSP1 cells was confirmed by Northern blot analysis. Fig. 3B:Tumor growth of SK-LMS cells and the influence of TSP-1 overexpression. SK-LMS-1 control cells, SK/HGF control cells and SK/HGF-TSP1 cells (clone 26) were subcutaneously implanted in *athymic nude* mice, respectively. The animals were monitored for tumor growth and tumor volumes (Tvol) were measured twice a week. The Tvol values represent an average of four mice for each group (P<0.025). Fig 3C: Visualization of the tumors at sacrifice. Fig. 3D: TSP-1 protein in tumor xenografts is derived from SK/HGF-TSP1 cells. Cell extracts were prepared from fresh tumors and TSP-1 protein was detected by anti-TSP-1 antibody under denatured condition.

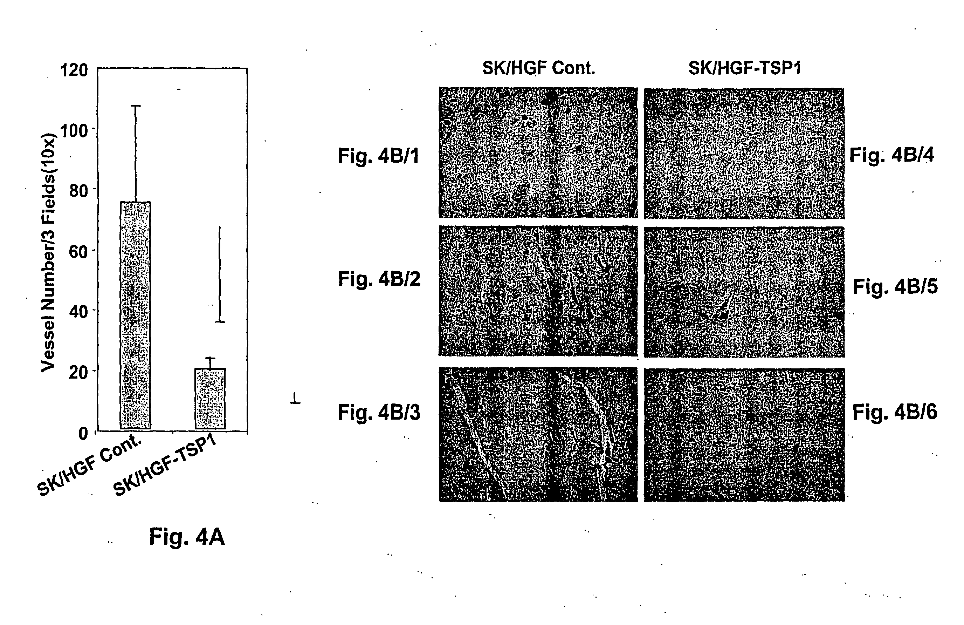

Fig. 4A and 4B/1-4B/6 are a graph and photomicrographs showing that TSP-1 inhibits HGF/SF-induced tumor angiogenesis. Fig. 4A: Decreased neovascularization in SK/HGF-TSP1 tumors: Tissue sections prepared from tumors derived from SK/HGF and SK/HGF-TSP1 groups were immunohistochemically stained with anti-mouse CD31 antibody. Three fields (10x magnification) from each stained tumor section were photographed and the numbers of CD31-positive vessels (brown staining) were scored. The numbers represent the average number of blood vessels in sections from four tumors for each group (P<0.01). In Fig. 3B, three representative fields from each group of tumors are displayed (Fig. 3B/1-3 are SK-HGF; Figs 3B/4-6 are SK/HGF-TSP1. Arrows indicate the CD31-positive vessels.

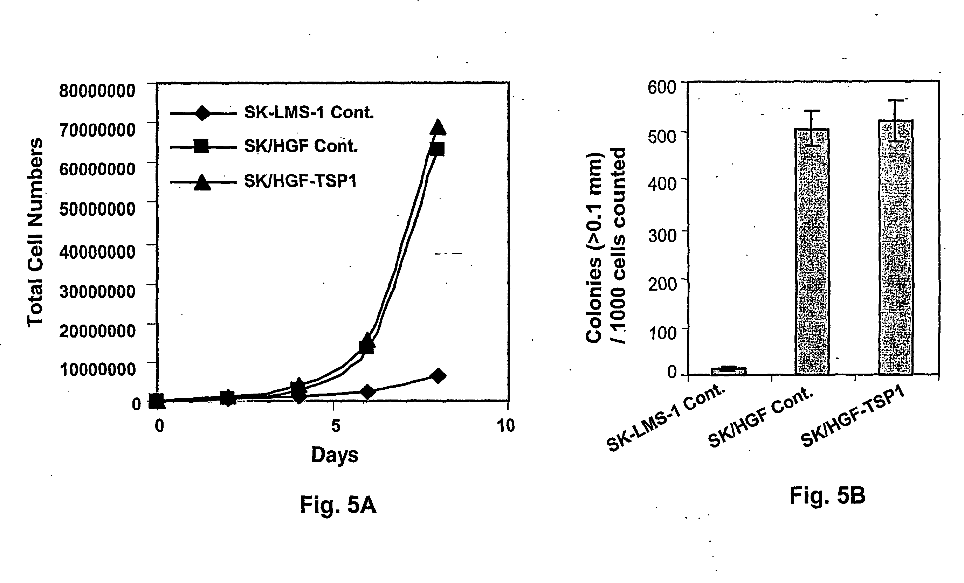

Figures 5A and 5B are graphs showing that overexpression of TSP-1 has no effect on cell proliferation or anchorage-independent growth compared the parental SK/HGF cells *in vitro.*

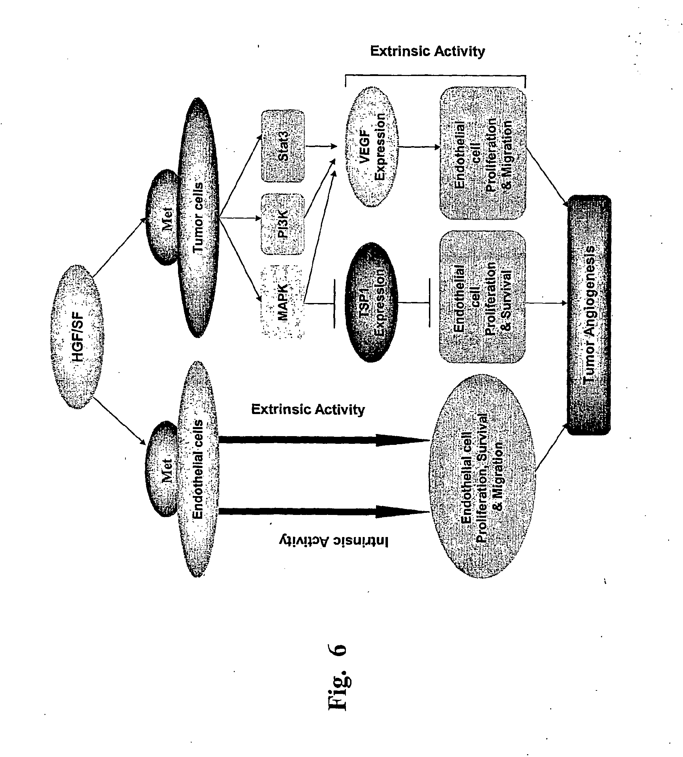

Figure 6 is a schematic representation of tumor angiogenesis induced by HGF/SF-Met signaling. Intrinsically, HGF/SF activates the Met receptor on the surface of the host endothelial cells, inducing proliferation and migration. Extrinsically, HGF/SF-Met signaling turns on the angiogenic switch by simultaneously up-regulating pro-angiogenic factor VEGF and down-regulating anti-angiogenic factor TSP-1 expression from the tumor cells, and thereby influences tumor angiogenesis. Interestingly, in the normal endothelial cells (HUVEC), we observed significant level of TSP-1 expression which can be down-regulated by HGF/SF-Met signaling, while the VEGF expression is undetectable.

## DESCRIPTION OF THE PREFERRED EMBODIMENTS

Targeting on angiogenesis is an effective way to prevent tumor development. Switching on or off tumor angiogenesis depends on the balance of pro-angiogenic and anti-angiogenic activities. Current efforts in preventing tumor angiogenesis are directed either to inhibition of pro-angiogenic activity such as with an anti-VEGF neutralizing antibody or to stimulation of anti-angiogenic activity by using an angiogenic inhibitor such as TSP-1 or a TSP-1 agonist. The present invention is based on the conception of targeting these two counter-balanced activities simultaneously by combination of these two approaches which can inhibit pro-angiogenic activity and increase anti-angiogenic activity. This approach should be a significant addition to our ability to intervene clinically in the process of tumor angiogenesis, through which it is possible to inhibit or prevent tumor malignancy.

The terms and abbreviations "hepatocyte growth factor," "HGF," "hepatocyte growth factor/scatter factor" and "HGF/SF" are used interchangeably and refer to a growth factor typically having a structure with six domains (finger, four Kringle regions (K1, K2, K3, K4) and serine protease domains). HGF has a heparin binding domain ("HBD") between the N-terminus and the K1 region. The mAbs and other HGF binding partners of the present invention may also bind to fragments of HGF and variants of HGF. The HGF molecules described herein include human HGF ( "huHGF") and homologues from any non-human mammalian species including mouse and rat HGF. The terms as used herein include mature, pre, pre-pro, and pro forms of the protein, and include polypeptides or peptides purified from a natural source, chemically synthesized or recombinantly produced. Human HGF is encoded by a cDNA sequence disclosed by Miyazawa et al., 1989, Biochem. Biophys. Res. Comm. 163:967-973), or Nakamura et al., 1989, Nature 342:440-443). The sequences reported by Miyazawa *et al.,* and Nakamura *et al.,* differ in 14 amino acids for reasons that are not understood and may be related to polymorphism or cloning artifacts. Both sequences are specifically encompassed herein. Natural allelic variations exist and can occur among individuals. HGF also includes the deltas5 huHGF as disclosed by Seki et al., 1989, Biochem Biophys. Res. Commun. 172:321-327 (1990)) and the variants disclosed by Rubin *et al.* (Proc Natl Acad Sci USA 88:415-419 (1991) and Science 254:1382-5 (1991).

The terms "HGF receptor" and "Met" refer to a cellular receptor for HGF, which typically includes an extracellular domain (ECD), a transmembrane domain ITMD) and an intracellular domain (ICD). Also included are variants and fragments of Met which retain the ability to bind HGF. The receptor may be the full-length polypeptide with the native amino acid sequence encoded by the gene known as p190. The present definition specifically encompasses soluble forms of HGF receptor, and HGF receptor from natural sources, synthetically produced or obtained by recombinant technology. HGF receptor variants include homologues which preferably share at least about 65% sequence identity, and preferably at least about 75% sequence identity, more preferably at least about 85% sequence identity, and most preferably at least about 95% sequence identity with any domain of the human Met amino acid sequence published in Rodrigues et al., Mol. Cell. Biol., 11:2962-2970 (1991); Park et al., Proc Natl Acad Sci USA 84:6379-6383 (1987); or Ponzetto et al., Oncogene 6:553-559 (1991).

## MAPK PATHWAY INHIBITORS

### MEK-Directed Proteases

One of the present inventors and colleagues observed in the National Cancer Institute's Antineoplastic Drug Screen (NCI-ADS) database (Koo, H.-M. et al., Canc Res 56:5211-5216 (1996); Monks, A. et al., JNatl Canc Inst 83: 757-766 (1991); Grever, M.R. et al., Sem Oncol 19:622-638 (1992)) that the lethal factor (LF) of Bacillus anthracis, a MEK-directed protease (Duesbery, N.S. et al., Science 280:734-737 (1998); Vitale, G. et al., Biochem Biophys Res Comm 248:706-711 (1998)) displayed enhanced tumor cell growth inhibition, in particular against melanoma lines.

The term "MEK-directed protease activity" refers the proteolytic activity of a protease on MEK1 resulting in inactivation of MEK1. This term is intended to include protease activity on any member of the MEK family. The designation MEK refers to a family of protein kinases that are part of the MAPK pathway. Examples are MEK1, MEK2 and MEK3, *etc.*). These proteins share sequence similarity, particularly at the N-terminus. See, for example, Duesbery, NS et al.,

CMLS Cell. Mol. Life Sci. 55:1599-1609 (1999).

Thus, a MEK-directed protease refers to

(1) a protease acting on members of the MEK protein family,

(2) a protease that acts on conservative amino acid substitution variants or other conservatively modified variants thereof; and

(3) a protease that acts on allelic or polymorphic variants, muteins and homologues in other species with greater than about 60%, preferably greater than about 70%, more preferably greater than about 80%, most preferably greater than about 90% sequence identity to MEK1, MEK2, MEK3, *etc.*

In one embodiment, MEK (*i.e.*, MEK1 and MEK2) is inhibited by *Bacillus anthracis* lethal factor (LF), a MEK-specific protease. LF is cytotoxic toward V12 H-ras-transformed NIH 3T3 cells and causes regression of MEK dependent tumor xenografts of these cells (Duesbery et al. Proc. Natl. Acad. Sci. USA 98: 4098-4094).

In another embodiment, the protease is a *Yersinia* protein, YopJ, and its homologues in other species and genera (avrRxv, Y4LO, AvrA), proteases that act on MEK1. LF, YopJ and their homologues, functional derivatives and mimetics are useful for inhibiting the MAPK pathway and contributing to the antitumor effects of the present combination of agents.

According to the present disclosure, the MEK (or homologue or mimetic) exerts is proteolytic action by recognizing a specific amino acid sequence present in MEK1 or in any member of the MEK family. Thus, methods described herein as targeting MEK1 can be carried out similarly without undue experimentation and with the same expected effect using an inhibitor active on any other MEK family member. Homologues of LF from other *Bacillus* species and mutants thereof that possess the characteristics disclosed herein are intended within the scope of this invention.

Also included is a "functional derivative" of LF, which is means an amino acid substitution variant, a "fragment," or a "chemical derivative" of LF, which terms are defined below. A functional derivative retains at least a portion of the relevant LF activity, that of proteolysis of MEK1 which permits its utility in accordance with the present invention.

With respect to the use of YopJ from *Yersinia pestis* or *Yersinia pseudotuberculosis*, it is to be understood that homologues of YopJ from other *Yersinia* species, and mutants thereof, that possess the characteristics disclosed herein are intended within the scope of this invention. Also included are "functional derivatives" of YopJ (as described above for LF).

A functional homologue must possess MEK-protease activity. In view of this functional requirement, use of homologous proteins to LF and YopJ from other bacterial species and genera, as well as from plant or animals sources, including proteins not yet discovered, fall within the scope of the invention if these proteins have sequence homology and the recited biochemical and biological activity.

It is within the skill in the art to obtain and express such a protein using DNA probes based on the sequence of LF or YopJ or *Salmonella*-derived or plant-derived homologues already characterized. Then, the protein's biochemical and biological activity can be tested readily using art-recognized methods such as those described herein, for example, a standard gel mobility shift assay for proteolysis of the substrate protein MEK1, or inhibition of MEK1-mediated phosphorylation of its natural substrate, MAPK, or of a model substrate. Finally, a biological assay of anti-melanoma activity where apoptosis or other measures of cytotoxic action of the protein are assessed, will indicate whether the homologue has the requisite activity to qualify as a functional homologue.

Similarly, for other polyeptides such as VEGF, TSP-1, *etc.*, and agonists and mimics thereof, assays for biological or biochemical activity for these molecules are well-known in the art, and it is within the skill of the art to test any such molecule to determine if it is a functional derivative or active variant, *etc.*, of the reference polypeptide.

A "variant" of the MEK-directed protease (or any other polypeptide of the present invention) refers to a molecule substantially identical to either the full protein or to a fragment thereof in which one or more amino acid residues have been replaced (substitution variant) or which has.one or several residues deleted (deletion variant) or added (addition variant). A "fragment" of the polypeptide, *e.g.*, the MEK-directed protease, is to any subset of the molecule, that is, a shorter polypeptide of the full length protein.

A preferred group of MEK-directed protease variants, or variants of other polypeptide molecules, are those in which at least one amino acid residue and preferably, only one, has been substituted by different residue. For a detailed description of protein chemistry and structure, see Schulz, GE et al., Pr-inciples of Protein Structure, Springer-Verlag, New York, 1978, and Creighton, T.E., Proteins: Structure and Molecular Properties, W.H. Freeman & Co., San Francisco, 1983, . The types of substitutions that may be made in the protein molecule may be based on analysis of the frequencies of amino acid changes between a homologous protein of different species, such as those presented in Table 1-2 of Schulz *et al. (supra)* and Figure 3-9 of Creighton (*supra*). Based on such an analysis, conservative substitutions are defined herein as exchanges within one of the following five groups:

| 1 | Small aliphatic, nonpolar or slightly polar residues | Ala, Ser, Thr (Pro, Gly); |
|---|---|---|
| 2 | Polar, negatively charged residues and their amides | Asp, Asn, Glu, Gln; |
| 3 | Polar, positively charged residues | His, Arg, Lys; |
| 4 | Large aliphatic, nonpolar residues | Met, Leu, Ile, Val (Cys) |
| 5 | Large aromatic residues | Phe, Tyr, Trp. |

The three amino acid residues in parentheses have special roles in protein architecture. Gly, the only residue lacking a side chain, imparts flexibility to the chain. Pro, because of its unusual geometry, tightly constrains the chain. Cys can participate in disulfide bond formation which is important in protein folding.

More substantial changes in biochemical, functional (or immunological) properties are made by selecting substitutions that are less conservative, such as between, rather than within, the above five groups. Such changes will differ more significantly in their effect on maintaining (a) the structure of the peptide backbone in the area of the substitution, for example, as a sheet or helical conformation, (b) the charge or hydrophobicity of the molecule at the target site, or (c) the bulk of the side chain. Examples of such substitutions are (i) substitution of Gly and/or Pro by another amino acid or deletion or insertion of Gly or Pro; (ii) substitution of a hydrophilic residue, *e.g.*, Ser or Thr, for (or by) a hydrophobic residue, *e.g.*, Leu, Ile, Phe, Val or Ala;

(iii) substitution of a Cys residue for (or by) any other residue; (iv) substitution of a residue having an electropositive side chain, *e.g.*, Lys, Arg or His, for (or by) a residue having an electronegative charge, *e.g.*, Glu or Asp; or (v) substitution of a residue having a bulky side chain, *e.g.*, Phe, for (or by) a residue not having such a side chain, *e.g.*, Gly.

Most acceptable deletions, insertions and substitutions according to the present invention are those that do not produce radical changes in the characteristics of the protein in terms of its proteolytic activity. However, when it is difficult to predict the exact effect of the substitution, deletion or insertion in advance of doing so, one skilled in the art will appreciate that the effect can be evaluated by routine screening assays such as those described here, without requiring undue experimentation.

Whereas shorter chain variants can be made by chemical synthesis, for the present invention, the preferred longer chain variants are typically made by site-specific mutagenesis of the nucleic acid encoding the polypeptide, expression of the variant nucleic acid in cell culture, and, optionally, purification of the polypeptide from the cell culture, for example, by immunoaffinity chromatography using specific antibody immobilized to a column (to absorb the variant by binding to at least one epitope).

The activity of a variant present in a cell lysate or a more highly purified preparation is screened in a suitable screening assay for the desired characteristic, preferably the proteolysis of MEK1. It is also possible to follow the immunological character of the protein molecule is assayed by alterations in binding to a given antibody, and may measured by competitive immunoassay. Biochemical or biological activity is screened in an appropriate assay, as described below.

A "mimetic" of a MEK-directed protease is an agent, generally a polypeptide or peptide molecule, or a peptidomimetic, that recognizes MEK, *e.g.*, MEK1, as a substrate and cleaves MEK1 at the same site cleaved by full-length, native protease such as LF or YopJ. Thus, such mimetics include homologues, peptides, conservative substitution variants, as well as deletion variants that retain the protease active site and proteolytic action on MEK1. Such mimetics are tested using assays for protease activity, *e.g.*, MEK1 mobility shift assays, MOS-induced activation of MAPK in oocytes and myelin basic protein (MBP) phosphorylation, as described below. In assessing a mimetic, LF is generally the positive control for protease activity. A mimetic has at least about 25% of the activity of this positive control, more preferably at least about 50-100% of the activity.

Similarly, mimetics of other polypeptides or peptides of this invention are molecules that express the activity of the polypeptide or peptide, bind to the same receptor with comparable affinity, and induce the same post-receptor binding intracellular pathway where appropriate.

Also useful in the present methods are agents that potentiate or promote the above proteolytic activity may be used along with LF or YopJ, their homologues or mimetics to promote their anti-tumor activity. A "potentiator" of the protease is an agent that activates (promotes, enhances, increases) the proteolytic activity and is identified by *in vitro* or *in vivo* assays of this activity or downstream activities in the MAPK pathway.

Samples that are treated with a candidate protease potentiator are compared to control samples that have not been treated with the test compound. This permits assessment of the presence and extent of activation of MEK1 protease activity. Control samples (untreated with test compounds) are assigned a relative protease activity value of 1. Activation is achieved when the measured protease activity value is about 1.5, more preferably 2.0 or greater. Potentiators can also be evaluated in a cellular assay, for example an assay for growth inhibition or apoptosis of human melanoma cells in culture as exemplified herein.

Fusion Proteins

The present disclosure utilizes a fusion protein comprising the MEK-directed protease (or homologue, functional derivative or mimetic) that is fused to another peptide or polypeptide that confers useful properties on the fusion protein.

One protein useful as a fusion partner is the domain of LF that binds to the protective antigen ("PA") of the anthrax toxin complex produced by *Bacillus anthracis* (Leppla, SH, "Anthrax Toxins," In: Handbook of Natural Toxins: Bacterial Toxins and Virulence Factors in Disease, Moss, J. et al., eds., Dekker, New York, 1995). For a recent review of anthrax toxins, see Duesbery, NS et al., CMLS Cell. Mol. Life Sci. 55:1599-1609 (1999). PA is one of three protein components of the "lethal" or "anthrax" toxin produced by *B. anthracis*. The 83kDa PA binds to a cell surface receptor present on almost all vertebrate cells, and its C-terminus is necessary for this binding (Singh, Y et al., J. Biol. Chem. 264:19103-19107 (1989); Novak, J. et al., J. Biol. Chem. 267:17186-17193 (1992)). After binding, PA is specifically cleaved by a protease (*e.g.*, furin, clostripain or trypsin), releasing a 20 kDa N-terminal PA fragment while a 63kDa C-terminal PA fragment (PA63) remains bound. PA63, also referred to as "processed PA," contains the receptor binding site at its C-terminus. PA63 forms a heptameric membrane- inserted channel which mediates the entry of the two other protein components of the complex (LF, and Edema factor, EF) into the cytosol via the endosomal pathway (Gordon et al., Infect. Immun. 56:1066-1069 (1988); Milne et al., J. Biol Chem. 269:20607-20612 (1994)).

To promote the uptake and processing of the MEK-directed protease (or homologue, derivative or mimetic), a fusion protein is made between the protease and the 250 amino acid PA-binding domain of LF. This will promote receptor binding and endosomal targeting of the fusion partner. As used herein, the term "PA" is a PA protein (or functional homologue or derivative) that has its receptor binding site intact and functional. US 5,591,631 and 5,677,274 describe PA fusion proteins that target PA to particular cells, such as cancer cells, using, as fusion partners, ligands for receptors on the targeted cells. In contrast, the present disclosure exploits the receptor-binding properties of PA by creating fusion proteins between the MEK-directed protease and the PA-binding domain of LF. The LF domain can be fused at the N- or C-terminus of the protease. The full length MEK-directed protease is not required in this fusion protein as long as the domain(s) responsible for the protease activity is (are) present. Such fusion proteins have the advantage of facilitating the uptake of the proteolytic polypeptide into the endosomal compartment and ultimately into the cytoplasm of the cell being targeted.

Chemical Modification of the Protein

A "chemical derivative" of a MEK-directed protease, or of another polypeptide of the present disclosure, contains additional chemical moieties not normally a part of the protein. Covalent modifications of the protein are included within the scope of this disclosure. Such modifications may be introduced into the molecule by reacting targeted amino acid residues with an organic derivatizing agent that is capable of reacting with selected side chains or terminal residues. Such chemically modified and derivatized moieties may improve the protein's solubility, absorption, biological half life, and the like. These changes may eliminate or attenuate undesirable side effects of the protein *in vivo*. Moieties capable of mediating such effects are disclosed, for example, in Remington's Pharmaceutical Sciences, Mack Publishing Company, Easton Pennsylvania (Gennaro 18th ed. 1990).

As noted above, TSP-1 agonists include TSP-1 homologues, functional derivatives, including fusion proteins and peptides, and other mimetics of TSP-1, as well as chemically modified TSP-1 proteins and peptides, as defined above for MEK protease homologues, *etc.*

**Preparation of Recombinant Proteins**

As described herein, native for recombinant MEK-directed protease proteins and TSP-1 agonist proteins, their homologues and mimetics are used in the methods of the disclosure. MEK1, the target of proteolytic activity, may also be provided in native or recombinant form for testing. Recombinant proteins may be particularly convenient for biochemical assays. MEK-directed protease and TSP-1 homologues and functional derivatives such as substitution variants and fusion proteins may be prepared recombinantly for evaluation of their mimetic activity and therapeutic activity. Recombinant proteins are prepared by conventional means which as a biochemically isolated and purified proteins from natural sources.

**Small Molecule Inhibitors of MEK**

Also intended within the scope of this disclosure are small organic molecules that act as MAPK inhibitors, such as inhibitors of MEK. As used herein, "small molecules" are organic chemical entities that are not biological macromolecules such as proteins or peptides. The small molecule inhibitors of MEK generally have a molecular mass of less than about 2000 D, preferably less than about 1000 D, more preferably less than about 500 D.

In a preferred embodiment, inhibition of MEK by the small molecule inhibitor PD98059 results in the efficient induction of apoptosis in cells of a human melanoma cell line.

Other small molecule inhibitors of the MAPK pathway are known to be, or are expected to be, cytotoxic to melanoma cells. These include the MEK inhibitors PD184352 (from Pfizer, originally Parlce-Davis) (Sebolt-Leopold, JS et al., Nature Med. 5: 810-816 (1999)), PD98059 (Dudley, D.T. et al., Proc Nat'l Acad Sci USA 92:7686-7689 (1995); Alessi, D.R. et al., J Biol Chem 270:27489-27494 (1995)) and U0126 (DuPont) (Favata, M et al., J Biol. Chem. 273: 18623-18632 (1998)), the p38 kinase inhibitor SB 203580 (Schering-Plough) (Cuenda, A et al., FEBS Lett. 364:229-233 (1995)), and the like.

VEGF-Trap

Wong AK et al., Proc Natl Acad Sci USA 7481-7486 (2001) described a potent VEGF antagonist (VEGF-TRAP (R1R2) that after systemic administration, reduced the severity of an VEGF -induced hepatitis-like syndrome. This antagonist is a soluble combined truncated form of the fms-like tyrosine kinase (Flt) and kinase insert domain-containing receptor (KDR) receptor fused to IgG (See, also Wulff C et al., Endocrinology 143:2797-807 (2002). Holash, J et al. (Proc Natl Acad Sci USA. 99:11393-11398 (2002)) further described VEGF-Trap, a VEGF blocker with potent antitumor effects. One of the most effective ways to block the VEGF-signaling pathway is to prevent VEGF from binding to its normal receptors by administering decoy-soluble receptors. According to Holash *et al.*, the highest-affinity VEGF blocker described to date is a soluble decoy receptor created by fusing the first three Ig domains of VEGF receptor-1 to an Ig constant region; however, this fusion protein has very poor *in vivo* pharmacokinetic properties. By determining the requirements to maintain high affinity while extending *in vivo* half life, we were able to engineer a very potent high-affinity VEGF blocker that has markedly enhanced pharmacokinetic properties. This VEGF-Trap effectively suppressed tumor growth and vascularization *in vivo,* resulting in stunted and almost completely avascular tumors. VEGF-Trap-mediated blockade may be superior to that achieved by other agents, such as mAbs targeted against the VEGF receptor.

Huang J et al., Proc Natl Acad Sci USA 100:7785-90 (2003) (see also, Proc Natl Acad Sci USA. 100:8624-5 (2003) for comment) described regression of established tumors and metastases by potent VEGF blockade with VEGF Trap which abolished mature, preexisting vasculature in established xenografts, which was followed by tumor regression (including lung micrometastases). Potent blockade was said to be a potential new therapeutic option for patients with bulky, metastatic cancers.

In view of the foregoing, the present invention includes the use of VEGF Trap as one anti-VEGF agent used in combination with other agents, as described, to inhibit angiogenesis and tumor growth and metastasis.

**siRNAs**

Referenced is the disclosure of commonly assigned U.S. Provisional Application Serial No. 60/556. 773, filed 26- March 2004.

siRNAs suppress gene expression through a highly regulated enzyme-mediated process called RNA interference (RNAi) (Sharp, P.A., Genes Dev. 15:485-490 (2001); Bernstein, E et al., Nature 409:363-366 (2001); Nykanen, A et al., Cell 107:309-321 (2001); Elbashir, S.M. et al., Genes Dev. 15:188-200 (2001)). RNAi involves multiple RNA-protein interactions characterized by four major steps: assembly of siRNA with the RNA-induced silencing complex (RISC), activation of the RISC, target recognition and target cleavage. These interactions may bias strand selection during siRNA-RISC assembly and activation, and contribute to the overall efficiency of RNAi (Khvorova, A et al., Cell 115:209-216 (2003); Schwarz, DS et al. 115:199-208 (2003)))

Two publications that describe preferred approaches and algorithms for selecting siRNA sequences are: Far, RK et al., Nuc Acids Res, 2003, 314417-4424 and Reynolds, A et al., Nature Biotech. 2004, 22:326-330. Far *et al.* suggests options for assessing target accessibility for siRNA and supports the design of active siRNA constructs. This approach can be automated, adapted to high throughput and is open to include additional parameters relevant to the biological activity of siRNA. To identify siRNA-specific features likely to Contribute to efficient processing at each of the steps pf RNAi noted above, Reynolds *et al.*, *supra* performed a systematic analysis of 180 siRNAs targeting the mRNA of two genes. Eight characteristics associated with siRNA functionality were identified: low G/C content, a bias towards low internal stability at the sense strand 3'-terminus, lack of inverted repeats, and sense strand base preferences (positions 3, 10, 13 and 19). Application of an algorithm incorporating all eight criteria significantly improves potent siRNA selection. This highlights the utility of rational design for selecting potent siRNAs that facilitate functional gene knockdown.

Candidate siRNA sequences against an intended target, for example, VEGF, the VEGF receptor (VEGF-R) , or human HGF or the HGF receptor (c-Met) are selected using a process that involves running a BLAST search against the sequence of the nucleic acid encoding the target molecule and selecting sequences that "survive" to ensure that these sequences will not be cross matched with any other genes.

siRNA sequences selected according to such a process and algorithm may be cloned into an expression plasmid and tested for their activity in abrogating VEGF, VEGF-R, HGF or Met function in expressing cells of the appropriate animal species. Those sequences that show RNAi activity are preferably recloned into a replication-defective human adenovirus serotype 5 (Ad5).

One reason for selection of this viral vector the high titer obtainable (in the range of $10^{10}$) and therefore the high multiplicities-of infection that can be attained. For example, infection with 100 infectious units/ cell ensures all cells are infected. Another advantage of this virus is the high susceptibility and infectivity and the host range (with respect to cell types). Even if expression is transient, cells can go through multiple replication cycles before activity, *e.g.*, Met activity, recovers (see Examples in U.S. Serial No. 60/556,473). Moreover, some tumors undergo apoptosis in response to expression of the present siRNAs, so that even transient expression is adequate to kill the cells.

Preferred anti-human Met constructs described are si-hMet-Ad5[221] which had the strongest effects on human glioblastoma cells (using the line DBTRG as an example), human prostate cancer cells (using PC-3 as an example) and human gastric cancer cells (using MKN45 as an example).

Preferred viral vectors are those with prolonged suppressive effect against the target polypeptide, lasting beyond passage of the cells in culture.

In a most preferred embodiment, the inhibitory molecule is a double stranded nucleic acid (preferably an RNA), used in a method of RNA interference. RNA interference is the sequence-specific degradation of homologues in an mRNA of a targeting sequence in a siNA (small, or short, interfering nucleic acid, which term is meant to be equivalent to other terms used to describe nucleic acid molecules that are capable of mediating sequence specific RNAi (RNA interference), for example short (or small) interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically-modified siRNA, post-transcriptional gene silencing RNA (ptgsRNA), translational silencing, and others. Long double stranded interfering RNAs, such a miRNAs, appear to tolerate mismatches more readily than do short double stranded RNAs. In addition, as used herein, the term RNAi is meant to be equivalent to other terms used to describe sequence specific RNA interference, such as post transcriptional gene silencing, or an epigenetic phenomenon. For example, siNA molecules of the invention can be used to epigenetically silence genes at both the post-transcriptional level or the pre-transcriptional level. In a non-limiting example, epigenetic regulation of gene expression by siNA molecules of the invention can result from siNA mediated modification of chromatin structure and thereby alter gene expression (see, for example, Allshire (2002) Science 297, 1818-1819; Volpe et al. (2002) Science 297, 1833-1837; Jenuwein (2002) Science 297, 2215-2218*;* and Hall et al. (2002) Science 297, 2232-2237.)

An siNA can be designed to target any region of the coding or non-coding sequence of an mRNA. An siNA is a double-stranded polynucleotide molecule comprising self-complementary sense and antisense regions, wherein the antisense region comprises nucleotide sequence that is complementary to nucleotide sequence in a target nucleic acid molecule or a portion thereof and the sense region has a nucleotide sequence corresponding to the target nucleic acid sequence or a portion thereof. The siNA can be assembled from two separate oligonucleotides, where one strand is the sense strand and the other is the antisense strand, wherein the antisense and sense strands are self-complementary. The siNA can be assembled from a single oligonucleotide, where the self-complementary sense and antisense regions of the siNA are linked by means of a nucleic acid based or non-nucleic acid-based linker(s). The siNA can be a polynucleotide with a hairpin secondary structure, having self-complementary sense and antisense regions. The siNA can be a circular single-stranded polynucleotide having two or more loop structures and a stem comprising self-complementary sense and antisense regions, wherein the circular polynucleotide can be processed either *in vivo* or *in vitro* to generate an active siNA molecule capable of mediating RNAi. The siNA can also comprise a single stranded polynucleotide having nucleotide sequence complementary to nucleotide sequence in a target nucleic acid molecule or a portion thereof (or can be an siNA molecule that does not require the presence within the siNA molecule of nucleotide sequence corresponding to the target nucleic acid sequence or a portion thereof), wherein the single stranded polynucleotide can further comprise a terminal phosphate group, such as a 5'-phosphate (see for example Martinez et al. (2002) Cell 110, 563-574 and Schwarz et al. (2002) Molecular Cell 10, 537-568), or 5',3'-diphosphate.

In certain embodiments, the siNA molecule of the invention comprises separate sense and antisense sequences or regions, wherein the sense and antisense regions are covalently linked by nucleotide or non-nucleotide linkers molecules as is known in the art, or are alternately non-covalently linked by ionic interactions, hydrogen bonding, Van der Waals' interactions, hydrophobic interactions, and/or stacking interactions. Some preferred siRNAs are discussed in the Examples.

As used herein, siNA molecules need not be limited to those molecules containing only RNA, but further encompasses chemically-modified nucleotides and non-nucleotides. In certain embodiments, the short interfering nucleic acid molecules of the invention lack 2'-hydroxy (2'-OH) containing nucleotides. In certain embodiments, short interfering nucleic acids do not require the presence of nucleotides having a 2'-hydroxy group for mediating RNAi and as such, short interfering nucleic acid molecules of the invention optionally do not include any ribonucleotides (*e.g.*, nucleotides having a 2'-OH group). Such siNA molecules that do not require the presence of ribonucleotides within the siNA molecule to support RNAi can however have an attached linker or linkers or other attached or associated groups, moieties, or chains containing one or more nucleotides with 2'-OH groups. Optionally, siNA molecules can comprise ribonucleotides at about 5, 10, 20, 30, 40, or 50% of the nucleotide positions. The modified short interfering nucleic acid molecules of the invention can also be referred to as short interfering modified oligonucleotides "siMON." Other chemical modifications, *e.g.*, as described in PCT/US03/05346 and PCT/US03/05028, can be applied to any siNA sequence of the invention.

Preferably a molecule mediating RNAi has a 2 nucleotide 3' overhang. If the RNAi molecule is expressed in a cell from a construct, for example from a hairpin molecule or from an inverted repeat of the desired sequence, then the endogenous cellular machinery will create the overhangs.

Considerations to be taken into account when designing an RNAi molecule include, *e.g.*, the sequence to be targeted, secondary structure of the RNA target and binding of RNA binding proteins. Methods of optimizing siRNA sequences will be evident to the skilled worker. Typical algorithms and methods are described, *e.g.*, in Vickers et al. (2003) J Biol Chem 278, 7108-7118; Yang et al. (2003) Proc Natl Acad Sci USA 99, 9942-9947; Far et al. (2003) Nuc. Acids Res. 31, 4417-4424; and Reynolds et al. (2004) Nature Biotechnology 22, 326-330.

Methods of making siRNAs are conventional. *In vitro* methods include processing the polyribonucleotide sequence in a cell-free system (*e.g.*, digesting long dsRNAs with RNAse III or Dicer), transcribing recombinant double stranded DNA *in vitro,* and, preferably, chemical synthesis of nucleotide sequences homologous to cMet sequence. See, *e.g.*, Tuschl et al. (1999) Genes & Dev. 13, 3191-3197.

*In vivo* methods include

(1) transfecting DNA vectors into a cell such that a substrate is converted into siRNA *in vivo* [see, *e.g.*, Kawasaki et al. (2003) Nucleic Acids Res 31, 700-707; Miyagishi et al. (2003) Nature Biotechnol 20, 497-500; Lee et al. (2002) Nature Biotechnol 20, 500-505, Brummelkamp et al. (2002) Science 296, 550-553; McManus et al. (2002) RNA 8, 842-850; Paddison et al. (2002a) Gene Dev 16, 948-958; Paddison et al. (2002b) Proc Natl Acad Sci USA 99, 1443-1448); Paul et al. (2002) Nature Biotechnol 20, 505-508; Sui et al. (2002) Proc Natl Acad Sci USA 99, 5515-5520; Yu et al. (2002) Proc Natl Acad Sci USA 99, 6047-6052];
(2) expressing short hairpin RNAs from plasmid systems using RNA polymerase III (pol III) promoters [see, *e.g.*, Kawasaki *et al.*, *supra;* Miyagishi *et al., supra*; Lee *et al., supra*; Brummelkamp *et al.*, *supra*; McManus *et al.*, *supra*), Paddison *et al.,* 2002a, 2002b, *supra*, Paul *et al., supra*, Sui *et al., supra*; and Yu *et al., supra*]; and/or
(3) expressing short RNA from tandem promoters [see, *e.g.*, Miyagishi *et al., supra*; and Lee *et al., supra*)].

When synthesized *in vitro*, a typical $\mu$M scale RNA synthesis provides about 1 mg of siRNA, which is sufficient for about 1000 transfection experiments using a 24-well tissue culture plate format. In general, to inhibit cMet expression in cells in culture, one or more siRNAs can be added to cells in culture media, typically at about 1 ng/ml to about 10 $\mu$g siRNA/ml.

For reviews on inhibitory RNAs, see *e.g.,* Lau et al. (2003) Scientific American, pp. 34-41; McManus et al. (2002) Nature Reviews Genetics 3, 737-747; and Dykxhoorn et al. (2003) Nature Reviews Molecular Cell Biology 4, 457-467. For further guidance regarding methods of designing and preparing siRNAs, testing them for efficacy, and using them in methods of RNA interference (both *in vitro* and *in vivo*), see, *e.g.,* Allshire (2002) Science 297, 1818-1819; Volpe et al. (2002) Science 297, 1833-1837; Jenuwein (2002) Science 297, 2215-2218; Hall et al. (2002) Science 297 2232-2237; Hutvagner et al. (2002) Science 297, 2056-60; McManus et al. (2002) RNA 8, 842-850; Reinhart et al. (2002) Gene & Dev. 16, 1616-1626; Reinhart et al. (2002) Science 297, 1831; Fire et al. (1998) Nature 391, 806-811, Moss (2001) Curr Biol 11, R772-5, Brummelkamp et al. (2002) Science 296, 550-3; Bass (2001) Nature 411 428-429; and Elbashir et al. (2001) Nature 411, 494-498; U.S. Pat 6,506,559; U.S. patent application 20030206887; and International patent publications WO99/07409, WO99/32619, WO 00/01846, WO 00/44914, WO00/44895, WO01/29058, WO01/36646, WO01/75164, WO01/92513, WO 01/29058, WO01/89304, WO01/90401, WO02/16620, and WO02/29858.

Ribozymes and siNAs can take any of the forms, including modified versions, described for antisense nucleic acid molecules; and they can be introduced into cells as oligonucleotides (single or double stranded), or in an expression vector.

In a preferred embodiment, an antisense nucleic acid, siNA (*e.g.*, siRNA) or ribozyme comprises a single stranded polynucleotide comprising a sequence that is at least about 90% (*e.g.*, at least about 93%, 95%, 97%, 98% or 99%) identical to a segment of the sequence of the target nucleic acid or a complement thereof. As used herein, a DNA and an RNA encoded by it are said to contain the same "sequence," taking into account that the thymine bases in DNA are replaced by uracil bases in RNA.

Active variants (*e.g.*, length variants, including fragments; and sequence variants) of the nucleic acid-based inhibitors discussed above are included in the invention. An "active" variant is one that retains an activity of the inhibitor from which it is derived (preferably the ability to inhibit expression)). A skilled worker can readily test a variant to determine if it is active, using conventional procedures.

With regard to length variants, an antisense nucleic acid or siRNA may be of any length that is effective for inhibition of a gene of interest. Typically, an antisense nucleic acid is between about 6 and about 50 nucleotides (*e.g.*, at least about 12, 15, 20, 25, 30, 35, 40, 45 or 50 nt), and may be as long as about 100 to about 200 nucleotides or more. Antisense nucleic acids having about the same length as the gene or coding sequence to be inhibited may be used. The length of an effective siNA is generally between about 15 bp and about 29 bp in length, preferably between about 19 bp and about 29 bp (*e.g.*, about 15, 17, 19, 21, 23, 25, 29 or 29 bp), with shorter and longer sequences being acceptable. Generally, siNAs are shorter than about 30 bp, to prevent eliciting interferon effects. For example, an active variant of an siRNA having, for one of its strands, the 19 nucleotide sequences disclosed in US. Serial no. 556,473 can lack base pairs from either, or both, of the ends of the double stranded RNA; or can comprise additional base pairs at either, or both, ends of the double stranded RNA, provided that the total of length of the siRNA is between about 19 and about 29 bp, inclusive.

As for sequence variants, it is generally preferable that an inhibitory nucleic acid, whether an antisense molecule, a ribozyme (the recognition sequences), or an siNA, comprises a strand that is complementary (100% identical in sequence) to a sequence of a gene that it is designed to inhibit. However, 100% sequence identity between the nucleic acid and the target gene is not required to practice the present invention. Thus, the invention has the advantage of being able to tolerate naturally occurring sequence variations, for example, in human c-met, that might be expected due to genetic mutation, strain polymorphism, or evolutionary divergence. Alternatively, the variant sequences may be artificially generated. Nucleic acid sequences with, *e.g.*, small insertions, deletions, and single point mutations relative to the target sequence can be effective for inhibition.

The degree of sequence identity may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). At least about 90% sequence identity (*e.g.*, at least about 92%, 95%, 98% or 99%), or even 100% sequence identity, between the inhibitory nucleic acid and the targeted sequence of the gene being silenced is preferred.

Alternatively, an active variant of an inhibitory nucleic acid of the invention is one that hybridizes to the sequence it is intended to inhibit under conditions of high stringency. For example, the duplex region of an siRNA may be defined functionally as a nucleotide sequence that is capable of hybridizing with a portion of the target gene transcript under high stringency conditions (*e.g.*, 400 mM NaCl, 40 mM PIPES pH 6.4, 1 mM EDTA, 50°C. or 70°C. hybridization for 12-16 hours), followed generally by washing.

Agents Targeting HGF/SF-Met Pathway

Agents with therapeutic potential to be used in the combinations of the present invention that target the HGF/SF-Met pathway include:

> (1) neutralizing antibody against human HGF/SF (Cao, B et al., Proc. Natl. Acad. Sci. 98:7443-7448, 2001; Int'l Patent Pub. WO 01/34650A1);
> (2) NK4, an antagonist of HGF/SF (Date, K. et al., Oncogene 17:3045-3054, 1998);
> (3) ribozymes targeting on HGF/SF and Met (Abounader, R et al., FASEB J. 16:108-110, 2002); and
> (4) other small molecule drugs (Webb, CP et al., Cancer Res. 60:342-349, 2000; Atabey, N et al., J. Biol. Chem. 276:14308-14314, 2001; Christensen, JG et al., Cancer Res., 63:7345-7355 2003)

Anti-HGF antibodies

B. Cao *et al*, *supra* disclosed that particular combinations of anti-HGF/SF mAbs could inhibit HGF/SF activity. This combination included three or more of the following anti-HGF/SF antibodies:

> (i) A.1, produced by hybridoma 1C10-F1-A11, ATCC # PTA3414;
> (ii) A.5, produced by hybridoma 13B1-E4-E10, ATCC# PTA3416;
> (iii) A.7, produced by hybridoma 15D7-B2, ATCC# PTA3413; and
> (iv) A.10, produced by hybridoma 31D4-C9-D, ATCC# PTA3412.

A particularly potent inhibitory combination is heparin with A.5, A.7 and A.10.

Cao *et al.*, *supra* described the preparation of these mAbs to human HGF/SF, by immunizing mice with native or denatured preparations of the ligand. Recloned mAbs were tested *in vitro* for blocking activity in bioassays of scattering and branching morphogenesis. The results showed that no single mAb was capable of neutralizing the *in vitro* activity of HGF/SF, and that the ligand possessed a minimum of three epitopes that must be blocked concurrently to prevent Met tyrosine kinase activation. *In vivo*, the neutralizing mAb combination inhibited subcutaneous (s.c.) growth in athymic *nu/nu* mice of tumors that depend on an autocrine Met-HGF/SF loop. Importantly, growth of human GBM xenografts expressing Met and HGF/SF was markedly reduced in the presence of anti HGF/SF-neutralizing mAb combinations. These results suggest interrupting autocrine and/or paracrine Met-HGF/SF signaling in tumors that depend on this pathway is a possible intervention strategy.

Anti-Met Antibodies

Another class of agents that can be used are antibodies specific for the Met receptor, preferably the human Met receptor. A number of publications disclose anti-Met antibodies. US Patents 5,686,292, 6,207,152, 6,214,344 to Schwall et al. disclose mAbs, particularly monovalent antibodies that are antagonists of the HGF receptor and their uses in treating cancer. US Patent 6,099,841 (Hillan et al.) discloses antibodies and fragments that are HGF receptor agonists. The document discloses that these molecules can be employed to substantially enhance HGF receptor activation, may be included in pharmaceutical compositions, articles of manufacture, or kits. Methods of treatment and *in vitro* diagnosis using these molecules HGF receptor agonists are also disclosed.

Prat et al., Mol Cell Biol 11:5954-5962 (1991) described several mAbs specific for the extracellular domain of the β-chain encoded by the c-Met gene (see also, WO 92/20792). The mAbs were selected following immunization of mice with whole live GTL-16 cells (human gastric carcinoma cell line) overexpressing Met. Four mAbs referred to as DL-21, DN-30, DN-31 and DO-24, were selected. Prat et al., Int J Canc 49:323-328 (1991) described using anti-c-Met mAb to detect distribution of the Met protein in human normal and neoplastic tissues. See, also, Yamada et al., Brain Res 637:308-312 (1994). The mAb DO-24 was reported to be an IgG2a isotype antibody.

Crepaldi et al., J Cell Biol 125:313-320 (1994) reported using mAbs DO-24 and DN-30 (*supra*) and mAb DQ-13 to identify subcellular distribution of HGF receptors in epithelial tissues and in MDCK cell monolayers. According to this document, DQ-13 was raised against a peptide corresponding to 19 C-terminal amino acids (from $Ser^{1372}$ to $Ser^{1390}$) of human c-Met.

A mAb specific for the cytoplasmic domain of human c-Met was described by Bottaro et al., Science 251: 801-804 (1991).

Silvagno et al., Arterioscler Thromb Vasc Biol 15:1857-1865 (1995) described use of a Met agonist antibody *in vivo* to promote angiogenesis in Matrigel® plugs.

According to Hillan *et al.*, *supra*; several of the mAbs cited above were commercially available from Upstate Biotechnology Incorporated, Lake Placid, NY (DO-24 and DL-21, specific for an extracellular epitope and DQ-13 specific for an intracellular epitope).

Cao and other colleagues of the present inventors raised and characterized mAbs against the extracellular domain of human Met:

(1) Met3 is produced by Hybridoma 2F6-B7-A11, (also referred to as "2F6") and has the Isotype: IgG2b/κ, and is deposited in the ATCC under Accession No. PTA-4349.

(2) Met5 is produced by Hybridoma 3A11-A8 (also referred to as "3A11") and is deposited in the ATCC under Accession No. PTA-4477.

A "monoclonal antibody or mAb" as used herein refers to an antibody that is part of a substantially, if not totally, homogeneous population of antibodies that are a product of a single B lymphocyte clone. mAbs are well known in the art and are made using conventional methods; see for example, Kohler and Milstein, Nature 256:495-497 (1975); U.S. Patent No. 4,376,110; Harlow, E. et al., Antibodies: A Laboratory Manual, Cold Spring Harbor Laboratory Press, Cold Spring Harbor, NY, 1988); Monoclonal Antibodies and Hybridomas: A New Dimension in Biological Analyses, Plenum Press, New York, NY (1980); H. Zola et al., in Monoclonal Hybridoma Antibodies: Techniques and Applications, CRC Press, 1982). mAbs may be produced recombinantly as well, *e.g.*, according to U.S. Pat. No. 4.816,567. mAbs may be derived from a single species, *e.g.*, a murine mAb or a human mAb, or may be chimeric.

The mAbs of the present invention are intended to include "chimeric" antibodies. A chimeric antibody is an Ig molecule wherein different parts of the molecule are derived from different animal species. An example is an Ig having a variable region derived from a murine mAb and a human Ig constant region. Also intended are antigen-binding fragments such chimeric antibodies. Chimeric antibodies and methods for their production are known in the art. See, for example, Cabilly et al, Proc. Natl. Acad. Sci. USA 81:3273-3277 (1984); Cabilly et al., U.S. Patents 4,816,567 (3/28/89) and 6,331,415 (12/18/01); Morrison et al., Proc. Natl. Acad. Sci. USA 81:6851-6855 (1984); Boulianne et al., Nature 312: 643-646 (1984); Neuberger et al., Nature 314:268-270 (1985); Sahagan et al., J. Immunol. 137:1066-1074 (1986); Liu et al., Proc. Natl. Acad. Sci. USA 84:3439-3443 (1987); Better et al., Science 240:1041- 1043 (1988)).

Preferred chimeric antibodies are "humanized" antibodies. Methods for humanizing non-human antibodies are well known in the art. Humanized forms of non-human (*e.g.,*, murine) antibodies are chimeric Igs, chains or fragments thereof (such as Fv, Fab, Fab', *etc.*,) which include minimal sequence derived from the non-human Ig. In a preferred humanized antibody, a human Ig recipient antibody receives residues from a CDR non-human species (donor or import antibody, *e.g.*, mouse, rat, rabbit) replacing the recipient CDR with the donor CDR residues. In some instances, Fv framework residues of the human Ig may be replaced by corresponding non-human residues. Humanized antibodies may also comprise residues which are found neither in the recipient antibody nor in the imported CDR or framework sequences. In general. the humanized antibody will comprise substantially all of at least one, and typically two, V domains, in which all or substantially all of the CDR regions correspond to those of a non-human Ig and all or substantially all of the FR regions are those of the human Ig consensus sequence. The humanized antibody optimally also will comprise at least part of a human Ig C region (*e.g.*, Fc). See, Jones et al., Nature 321:522-525 (1986); Reichmann et al., Nature 332:323-327 (1988); Presta, Curr. Op. Struct. Biol, 2:593-596 (1992); Verhoeyen et al., Science, 239:1534-1536 (1988)); U.S. Pat. No. 4,816,567),

The choice of human V domains, ($V_H$ and $V_L$) to be used in making the humanized antibodies is important for reducing the antigenicity of the product when administered repeatedly to a human. According to the "best-fit" method, the sequence of the V domain of a rodent antibody is screened against the entire library of known human Variable domain sequences. The human sequence which is closest to that of the rodent is then accepted as the human FR for the humanized antibody (Sims et al., J. Immunol. 151:2296 (1993); Chothia et al., J. Mol. Biol. 196:901 (1987)]. Another method uses a particular FR derived from the consensus sequence of all human antibodies of a particular subgroup of L or H chains. The same FR may be used for several different humanized antibodies (Carter et al., Proc. Natl. Acad. Sci. USA 89:4285 (1992); Presta et al., J. Immunol. 151:2623-2632 (1993)).

It is important that humanized antibodies retain their (preferably high) binding affinity for the antigen and other favorable biological properties. To achieve this, humanized antibodies are designed by a process of analysis of the parental sequences and various conceptual humanized products using three dimensional (3D) models of the parental and humanized sequences. 3D Ig models are commonly available and are known to those skilled in the art. Available computer programs illustrate and display probable 3D conformational structures of selected candidate Ig sequences. Inspection of these displays permits analysis of the likely role of certain amino acid residues in the functional capacity of the candidate Ig sequence. In this way, FR residues can be selected and combined from the consensus and import sequence so that the desired antibody characteristic is achieved. In general, the CDR residues are directly and most substantially involved in influencing antigen binding (*e.g.*,, WO 94/04679).

For production of human antibodies, transgenic animals (*e.g.*,, mice) that are capable, upon immunization, of producing a full repertoire of human antibodies in the absence of endogenous Ig production can be employed. For example, the homozygous deletion of the antibody H chain joining region ($J_H$) gene in chimeric and germ-line mutant mice results in complete inhibition of endogenous antibody production. Transfer of the human germ-line Ig gene array into such germ-line mutant mice will result in the production of human antibodies upon antigen challenge (Jakobovits et al., Proc. Natl. Acad. Sci. USA 90:2551-255 (1993); Jakobovits et al. Nature, 362:255-258 (1993); Bruggermann et al., Year in Immunol. 7:33 (1993)).

Human antibodies can also be produced in phage display libraries (Hoogenboom et al., J. Mol. Biol. 222:381 (1991); Marks et al., J. Mol. Bio., 222:581 (1991)). The techniques of Cote *et al.* and Boerner *et al.* are also available for the preparation of human mAbs (Cole et al., Monoclonal Antibodies and Cancer Therapy, Alan R. Liss, p. 77 (1985) and Boerner et al., J. Immunol, 147:86-95 (1991).

Other types of chimeric molecules or fusion polypeptides involving the present mAb or antigen-binding fragments of domains thereof, include those designed for an extended *in vivo* half-life. This may include first identifying the sequence and conformation of a "salvage receptor" binding epitope of an Fc region of an IgG molecule. A "salvage receptor binding epitope" refers here to an epitope or fragment of the Fc region of an IgG molecule of any isotype contributes to increasing the *in vivo* half-life of the particular IgG molecule (when compared to other Ig classes). Once this epitope is identified, the sequence of the mAb is modified to include the sequence and conformation of the identified binding epitope. After the sequence is mutated, the chimera is tested for longer *in vivo* half-life compared to the unmodified Ig molecule or chain. If a longer half-life is not evident, the sequence is altered further to include the sequence and conformation of the identified binding epitope. Care is taken that the antigen-binding activity or other desired biological activity of this chimeric molecule is maintained. The salvage receptor binding epitope generally constitutes a region corresponding to all or part of one or two loops of a Fc domain; preferably this sequence is "grafted" in an analogous position in the anti-Met antibody fragment. Preferably, three or more residues from one or two loops of the Fc domain are transferred; more preferably, the epitope is taken from the IgG $CH_2$ domain and transferred to one or more of the $CH_1$, $CH_3$, or $V_H$ region of the anti-Met antibody. Alternatively, the epitope from the $CH_2$ domain is transferred to the $C_L$ or the $V_L$ domain of the anti-Met antibody fragment.

Another chimeric molecule intended herein comprises the antibody chain, *e.g.*, anti-r VEGF, anti-VEGF-R, anti-HGF or anti-Met antibody chain or fragment fused to an Ig constant domain or to an unrelated ( heterologous) polypeptide such as albumin. Such chimeras can be designed as monomers, homomultimers or heteromultimers, with heterodimers preferred.

In another embodiment, the chimera comprises an antibody fragment fused to albumin. Such chimeras may be constructed by inserting the entire coding region of albumin into a plasmid expression vector. The DNA encoding the antibody chain or fragment can be inserted 5' to the albumin coding sequence, along with an insert that encodes a linker, e.g., $Gly_4$ (Lu et al., FEBS Lett 356:56-59 (1994)). The chimera can be expressed in desired mammalian cells or yeast.

In general, these various chimeric molecules can be constructed in a fashion similar to more conventional chimeric antibodies in which a Variable domain from one antibody is substituted for the V domain of another antibody. For further details n preparing such antibody-nonantibody fusions, see, for example, Capon et al., Nature 337:525 (1989); Byrn et al., Nature, 344:667 (1990)

Diabodies are small antibody fragments with two antigen binding sites, which fragments comprise $V_H$ domain bonded to a $V_L$ domain in the same polypeptide chain ($V_H$-$V_L$). By using a linker that is too short to allow pairing between the two domains on the same chain, the domains are forced to pair with the complementary domains of another chain and create two antigen binding sites. Diabodies are described in further detail, for example, in EP404,097; WO 93/11161; and Hollinger et al., Proc. Natl. Acad. Sci, 90:6444-6448 (1993).

An anti-idiotypic (anti-Id) antibody is an antibody which recognizes unique determinants generally associated with the antigen-binding site of another antibody. An anti-Id antibody can be prepared by immunizing an animal of the same species and genetic type (e.g., mouse strain) as the source of the mAb with the mAb to which an anti-Id is being prepared. The immunized animal will recognize and respond to the idiotypic epitopes of the immunizing antibody by producing an antibody to these idiotypic determinants (the anti-Id antibody). The anti-Id antibody may also be used as an "immunogen" to induce an immune response in yet another animal, producing a so-called anti-anti-Id antibody. The anti-anti-Id may be epitopically identical to the original mAb which induced the anti-Id. Thus, by using antibodies to the idiotypic determinants of a mAb, it is possible to identify other clones expressing antibodies of identical specificity. Anti-Id mAbs thus have their own idiotypic epitopes, or "idiotopes" structurally similar to the epitope if interest, such as a Met epitope.

Antibody Functional Derivatives and Chemically Modified Antibodies

Chemical, including, covalent modifications of antibodies are within the scope of this invention. One type of modification is introduced into the molecule by reacting targeted amino acid residues with an organic derivatizing agent that is capable of reacting with selected side chains or the N- or C- terminal residues.

Derivatization with bifunctional agents is useful for crosslinking the antibody (or fragment or derivative) to a water-insoluble support matrix or surface for use in a purification method (described below). Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis (succinimidylpropionate), and bifunctional maleimides such as bis-N-maleimido-1,8-octane. Derivatizing agents such as methyl-3-[(p-azidophenyl)dithio]propioimidate create photoactivatable intermediates that can crosslink when irradiated with light. Reactive water-insoluble matrices such as cyanogen bromide-activated carbohydrates and the reactive substrates described in U.S. Pat. Nos. 3,969,287; 3,691,016; 4,195,128; 4,247,642; 4,229,537; and 4,330,440 are used in protein immobilization.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the a-amino groups of lysine, arginine, and histidine side chain (see, for example, T. E. Creighton, Proteins: Structure and Molecular Properties, W. H. Freeman & Co., San Francisco, (1983)), acetylation of the N-terminal amine, and amidation of any C-terminal carboxyl group. The modified forms of the residues fall within the scope of the present invention.

Also included herein are antibodies in which the native glycosylation pattern of the polypeptide have been altered. This means deletion of one or more carbohydrate moieties and/or adding one or more glycosylation sites that are not present in the native polypeptide chains. Protein glycosylation is typically N-linked (attached to an Asp side chain) or O-linked (attached to a hydroxyamino acid, most commonly Ser or Thr; possibly 5-hydroxyPro or 5-hydroxyLys). The tripeptide Asp-Z-Ser and Asp-Z-Thr (where Z is any amino acid but Pro) are recognition sequences for enzymatic attachment of the carbohydrate moiety to the Asp side chain. The presence of either of these sequences creates a potential N-glycosylation site. O-linked glycosylation usually involves binding of N-acetylgalactosamine, galactose, or xylose. Addition of glycosylation sites to the polypeptide may be accomplished by altering the native amino acid sequence to include e one or more of the above-described tripeptide sequences (for N-linked glycosylation sites) or addition of, or substitution by, one or more Serine or Threonine (for O-linked glycosylation sites). The amino acid sequence may be altered through changes at the DNA level, e.g., by mutating the DNA encoding the Ig polypeptide chain at preselected bases to generate codons that encode the desired amino acids. See, for example U.S. Pat. No. 5,364.934.

Chemical or enzymatic coupling of glycosides to the polypeptide may also be used. Depending on the coupling mode used, the sugar(s) may be attached to (a) Arginine and His, (b) free carboxyl groups, (c) free sulfhydryl groups such as those of Cys, (d) free hydroxyl groups such as those of Serine, Thr, or hydroxyPro, (e) aromatic residues such as those of Phe, Tyr, or Trp, or (f) the amide group of Gln. These methods are described in WO87/05330 (11 Sept 1987) and in Aplin et al., CRC Crit. Rev. Biochem., pp. 259-306 (1981).

Removal of existing carbohydrate moieties may be accomplished chemically or enzymatically or by mutational substitution of codons (as described above). Chemical deglycosylation is achieved, for example, by exposing the polypeptide to trifluoromethanesulfonic acid, or an equivalent compound cleaves most or all sugars except the linking sugar (N-acetylglucosamine or N-acetylgalactosamine), while leaving the polypeptide intact. See: Hakimuddin et al., Arch. Biochem. Biophys., 259:52 (1987); Edge et al., Anal. Biochem. 118:131 (1981). Any of a number of endo- and exo-glycosidases are used for enzymatic cleavage of carbohydrate moieties from polypeptides (Thotakura et al., Meth. Enzymol. 138:350 (1987)).

Glycosylation at potential glycosylation sites may be prevented by the use of the tunicamycin (Duskin et al., J Biol Chem, 257:3105 (1982) which blocks formation of N-glycosidic linkages.

Another type of chemical modification of the present antibodies comprises bonding to any one of a number of different nonproteinaceous polymers, such as polyethylene glycol (PEG), polypropylene glycol, or polyoxyalkylenes, in the manner described in U.S. Patents No. 4,640,835; 4,496,689; 4,301,144; 4,670,417; 4,791,192 and 4,179,337 and WO93/00109.

In addition to *in vivo* diagnostic and therapeutic uses, the antibodies or fragments of the present invention may be used to quantitatively or qualitatively detect the presence of Met in a cellular or other biological sample. For example, it may be desired to monitor the level of Met in the circulation or in the tissues of a subject receiving a therapeutic dose or form of the mAb. Thus, the antibodies (or fragments thereof) useful in the present invention may be employed histologically to detect the presence of Met-bearing tumor cells.

The present invention is directed in particular to a number of useful mAbs reactive against various epitopes of the VEGF, VEGF-R Met, of HGF or the Met-HGF complex, and mAbs specific for an epitope on the ECD of Met of VEGF-R.

The mAbs and combinations of the present invention, along with various names used for each mAb (some being abbreviations of longer designations) are shown in Table 1, below. The hybridomas producing these mAbs have been deposited in the American Type Culture Collection (ATCC) prior to the filing of the present application. Their ATCC Patent Deposit Designations (or accession numbers), are provided in Table 1.

## Pharmaceutical Compositions, Their Formulation and Use

A pharmaceutical composition according to this invention comprises (1) one or more VEGF inhibitors such as an anti-VEGF mAb, in combination with (2) a TSP-1 agonist, in any suitable formulation known in the art.

Pharmaceutical compositions within the scope of this invention include all compositions wherein the VEGF/MAPK inhibitor and TSP-1 agonist are contained in an amount effective to achieve their intended purpose. While individual needs vary, determination of optimal ranges of effective amounts of each component is within the skill of the art. Typical dosages comprise 0.1 to 100 mg/kg/body wt, though more preferred dosages are described for certain particular uses, below.

In addition to the pharmacologically active protein or small molecule, the pharmaceutical compositions may contain suitable pharmaceutically acceptable carriers comprising excipients and auxiliaries which facilitate processing of the active compounds into preparations which can be used pharmaceutically as is well known in the art. Suitable solutions for administration by injection or orally, may contain from about 0.01 to 99 percent, active compound(s) together with the excipient.

The pharmaceutical preparations of the present invention are manufactured in a manner which is known, for example, by means of conventional mixing, granulating, dissolving, or lyophilizing processes. Suitable excipients may include fillers binders, disintegrating agents, auxiliaries and stabilizers, all of which are known in the art. Suitable formulations for parenteral administration include aqueous solutions of the proteins in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension.

The compositions may be in the form of a lyophilized particulate material, a sterile or aseptically produced solution, a tablet, an ampule, etc. Vehicles, such as water (preferably buffered to a physiologically acceptable pH, as for example, in phosphate buffered saline) or other inert solid or liquid material such as normal saline or various buffers may be present. The particular vehicle is not critical, and those skilled in the art will know which vehicle to use for any particular utility described herein.

In general terms, a pharmaceutical composition is prepared by mixing, dissolving, binding or otherwise combining the polymer or polymeric conjugate of this invention with one or more water-insoluble or water-soluble aqueous or non-aqueous vehicles. If necessary, another suitable additive or adjuvant is included. It is imperative that the vehicle, carrier or excipient, as well as the conditions for formulating the composition are such that do not adversely affect the biological or pharmaceutical activity of the protein, peptide or small molecule.

## Subjects, Treatments Modes and Routes of Administration

The preferred animal subject of the present invention is a mammal. The invention is particularly useful in the treatment of human subjects. By the term "treating" is intended the administering to subjects an effective amount of a pharmaceutical composition comprising one or a combination of agents, that may be given separately or as a single combination drug, and includes (a) an anti-angiogenic factor or anti-angiogenic agonist; and an inhibitor of angiogenic protein or pathway. A preferred embodiment comprises, TSP-1 or another anti-angiogenic factor, and an inhibitors of VEGF. Treating includes administering the agent to subjects at risk for Met-expressing (or Met-negative) tumors, for metastasis of such tumors or for recurrent tumors developing prior to evidence of clinical disease, as well as subjects diagnosed with such tumors who have not yet been treated or who have been treated by other means, *e.g.*, surgery, conventional chemotherapy, and in whom tumor burden has been reduced even to the level of not being detectable. Thus, this invention is useful in preventing or inhibiting primary growth, recurrent growth or metastatic growth of tumors.

The pharmaceutical compositions of the present invention, wherein a VEGF inhibitor and TSP-1 or TSP-1 agonist are combined with pharmaceutically acceptable excipient or carrier, are administered by any means that achieve their intended purpose. Amounts and regimens for the administration of can be determined readily by those with ordinary skill in the clinical art of treating any of the particular diseases. Preferred amounts are described below.

In general, the present methods include administration, preferably injection or infusion, by parenteral routes, including subcutaneous (s.c.) intravenous (i.v.), intramuscular, intraperitoneal, intrathecal as well as transdermal, topical or inhalation routes. Also intended are enteral, including oral routes of administration.

A preferred route is by direct intratumoral injection. Alternatively, or concurrently, administration may be by the oral route, particularly for the small molecule agents. The dosage administered will be dependent upon the age, health, and weight of the recipient, kind of concurrent treatment, if any, frequency of treatment, and the nature of the effect desired.

In one treatment approach, the compounds and methods are applied in conjunction with surgery. Thus, an effective amount of the VEGF inhibitor in combination with a TSP-1 agonist is applied directly to the site of surgical removal of a tumor mass (whether primary or metastatic). This can be done by injection or "topical" application in an open surgical site or by injection after closure.

In a preferred embodiment, the specified amount of a VEGF inhibitor and a TSP-1 agonist, each preferably about 2-100 $\mu$g, is added to about 700 ml of human plasma that is diluted 1:1 with heparinized saline solution at room temperature. Human IgG in a concentration of 500 $\mu$g/dl (in the 700 ml total volume) may also be used. The solutions are allowed to stand for about 1 hour at room temperature. The solution container may then be attached directly to an iv infusion line and administered to the subject at a preferred rate of about 20 ml/min.

In another embodiment, the pharmaceutical composition is directly infused i.v. into a subject. The appropriate amount, preferably about 2-100 $\mu$g of each agent in the combination, is added to about 250 ml of heparinized saline solution and infused iv into patients at a rate of about 20 ml/min.

In the present method, the composition can be given one time but generally is administered six to twelve times (or even more, as is within the skill of the art to determine empirically). The treatments can be performed daily but are generally carried out every two to three days or as infrequently as once a week, depending on the beneficial and any toxic effects observed in the subject.

The pharmaceutical formulation for systemic administration according to the invention may be formulated for enteral, parenteral or topical administration, and all three types of formulation may be used simultaneously to achieve systemic administration of the active ingredient.

For lung instillation, aerosolized solutions are used. In a sprayable aerosol preparations, the active protein or small molecule agent may be in combination with a solid or liquid inert carrier material. This may also be packaged in a squeeze bottle or in admixture with a pressurized volatile, normally gaseous propellant. The aerosol preparations can contain solvents, buffers, surfactants, and antioxidants in addition to the protein of the invention.

For topical application, the therapeutic compounds of the present invention may be incorporated into topically applied vehicles such as salves or ointments, as a means for administering the active ingredient directly to the affected area. Scarification methods, known from studies of vaccination, can also be used. The carrier for the active agent may be either in sprayable or nonsprayable form. Non-sprayable forms can be semi-solid or solid forms comprising a carrier indigenous to topical application and having a dynamic viscosity preferably greater than that of water. Suitable formulations include, but are not limited to, solution, suspensions, emulsions, creams, ointments, powders, liniments, salves, and the like. If desired, these may be sterilized or mixed with auxiliary agents, *e.g.*, preservatives, stabilizers, wetting agents, buffers, or salts for influencing osmotic pressure and the like. Examples of preferred vehicles for non-sprayable topical preparations include ointment bases, e.g., polyethylene glycol-1000 (PEG-1000); conventional creams such as HEB cream; gels; as well as petroleum jelly and the like.

Other pharmaceutically acceptable carriers according to the present invention are liposomes, pharmaceutical compositions in which the active protein is contained either dispersed or variously present in corpuscles consisting of aqueous concentric layers adherent to lipidic layers. The active protein is preferably present in the aqueous layer and in the lipidic layer, inside or outside, or, in any event, in the non-homogeneous system generally known as a liposomic suspension.

The hydrophobic layer, or lipidic layer, generally, but not exclusively, comprises phospholipids such as lecithin and sphingomyelin, steroids such as cholesterol, more or less ionic surface active substances such as dicetylphosphate, stearylamine or phosphatidic acid, and/or other materials of a hydrophobic nature.

### *In Vivo* Study of Antitumor Effects

Animal Models of Human Tumors

The combination compositions of the present invention are tested for therapeutic efficacy in well established rodent models which are considered to be representative of a human tumor. The overall approach is described in detail in

1. Geran, R.I. et al., "Protocols for Screening Chemical Agents and Natural Products Against Animal Tumors and Other Biological Systems (Third Edition)", Canc. Chemother. Reports, Part 3, 3:1-112, and

2. Plowman, J. et al., In: B. Teicher, ed., Anticancer Drug Development Guide: Preclinical Screening, Clinical Trials and Approval, Part II: In Vivo Methods, Chapter 6, "Human Tumor Xenograft Models in NCI Drug Development," Humana Press Inc., Totowa, NJ, 1997.

Both these references are hereby incorporated by reference in their entirety.

### General Test Evaluation Procedures

The compositions described herein may be tested for therapeutic efficacy in several well established rodent models which are considered to be highly representative of a broad spectrum of human tumors. These approaches are described in detail in Geran *et al., supra.*

### A. Calculation of Mean Survival Time (MST)

MST (days) is calculated according to the formula:

$$\frac{S + AS_{(A-1)} - (B + 1)\,NT}{S_{(A-1)} - NT}$$

Day:    Day on which deaths are no longer considered due to drug toxicity. For example, with treatment starting on Day 1 for survival systems (such as L1210, P388, B16, 3LL, and W256): Day A=Day 6; Day B=Day beyond which control group survivors are considered "no-takes."

S:    If there are "no-takes" in the treated group, S is the sum from Day A through Day B. If there are no "no-takes" in the treated group, S is the sum of daily survivors from Day A onward.

S(A-1):    Number of survivors at the end of Day (A-1).

Example:    for 3LE21, S(A-1)=number of survivors on Day 5.

NT:    Number of "no-takes" according to the criteria given in Protocols 7.300 and 11.103.

### B. T/C Computed for all treated groups

$$T/C = \frac{MST \text{ of treated group}}{MST \text{ of control group}} \times 100$$

Treated group animals surviving beyond Day Bare eliminated from calculations (as follows):

| No. of survivors in treated group beyond Day B | Percent of "no-takes" in control group | Conclusion |
|---|---|---|
| 1 | Any percent | "no-take" |
| 2 | <10 | drug inhibition |
|  | ³10 | "no-takes" |
| ³3 | <15 | drug inhibitions |
|  | ³15 | "no-takes" |

Positive control compounds are not considered to have "no-takes" regardless of the number of "no-takes" in the control group. Thus, all survivors on Day B are used in the calculation of T/C for the positive control. Surviving animals are evaluated and recorded on the day of evaluation as "cures" or "no-takes."

## Calculation of Median Survival Time (MedST)

MedST is the median day of death for a test or control group. If deaths are arranged in chronological order of occurrence (assigning to survivors, on the final day of observation, a "day of death" equal to that day), the median day of death is a day selected so that one half of the animals died earlier and the other half died later or survived. If the total number of animals is odd, the median day of death is the day that the middle animal in the chronological arrangement died. If the total number of animals is even, the median is the arithmetical mean of the two middle values. Median survival time is computed on the basis of the entire population and there are no deletion of early deaths or survivors, with the following exception:

## C. Computation of βMedST From Survivors

If the total number of animals including survivors (N) is even, the MedST (days) (X+Y)/2, where X is the earlier day when the number of survivors is N/2, and Y is the earliest day when the number of survivors (N/2)-1. IfN is odd, the MedST (days) is X.

## D. Computation of MedST From Mortality Distribution

If the total number of animals including survivors (N) is even, the MedST (days) (X+Y)/2, where X is the earliest day when the cumulative number of deaths is N/2, and Y is the earliest day when the cumulative number of deaths is (N/2)+1. IfN is odd, the MedST (days) is X. "Cures" and "no-takes" in systems evaluated by MedST are based upon the day of evaluation. On the day of evaluation any survivor not considered a "no-take" is recorded as a "cure." Survivors on day of evaluation are recorded as "cures" or "no-takes," but not eliminated from the calculation.

## E. Calculation of Approximate Tumor Weight From Measurement of Tumor Diameters with Vernier Calipers

The use of diameter measurements (with Vernier calipers) for estimating treatment effectiveness on local tumor size permits retention of the animals for lifespan observations. When the tumor is implanted sc, tumor weight is estimated from tumor diameter measurements as follows. The resultant local tumor is considered a prolate ellipsoid with one long axis and two short axes. The two short axes are assumed to be equal. The longest diameter (length) and the shortest diameter (width) are measured with Vernier calipers. Assuming specific gravity is approximately 1.0, and Pi is about 3, the mass (in mg) is calculated by multiplying the length of the tumor by the width squared and dividing the product by two. Thus,

$$\text{Tumor weight (mg)} = \frac{\text{length (mm) x (width [mm])}^2}{2} \quad \text{or} \quad \frac{\text{L x (W)}^2}{2}$$

The reporting of tumor weights calculated in this way is acceptable inasmuch as the assumptions result in as much accuracy as the experimental method warrants.

## F. Calculation of Tumor Diameters

The effects of a drug on the local tumor diameter may be reported directly as tumor diameters without conversion to tumor weight. To assess tumor inhibition by comparing the tumor diameters of treated animals with the tumor diameters of control animals, the three diameters of a tumor are averaged (the long axis and the two short axes). A tumor diameter T/C of 75% or less indicates activity and a T/C of 75% is approximately equivalent to a tumor weight T/C of 42%.

## G. Calculation of Mean Tumor Weight From Individual Excised Tumors

The mean tumor weight is defined as the sum of the weights of individual excised tumors divided by the number of tumors. This calculation is modified according to the rules listed below regarding "no-takes." Small tumors weighing 39 mg or less in control mice or 99 mg or less in control rats, are regarded as "no-takes" and eliminated from the computations. In treated groups, such tumors are defined as "no-takes" or as true drug inhibitions according to the following rules:

| Percent of small tumors in treated group | percent of "no-takes" in control group | Action |
|---|---|---|
| ≤17 | Any percent | no-take; not used in calculations |
| 18-39 | <10 | drug inhibition; use in calculations |
| | ≥10 | no-takes; not used in calculations |
| ≥40 | <15 | drug inhibition; use in calculations |
| | ≥15 | Code all nontoxic tests "33" |

Positive control compounds are not considered to have "no-takes" regardless of the number of "no-takes" in the control group. Thus, the tumor weights of all surviving animals are used in the calculation of T/C for the positive control (T/C defined above) SDs of the mean control tumor weight are computed the factors in a table designed to estimate SD using the estimating factor for SD given the range (difference between highest and lowest observation). Biometrik Tables for Statisticians (Pearson ES, and Hartley HG, eds.) Cambridge Press, vol. 1, table 22, p. 165.

## II. SPECIFIC TUMOR MODELS

### A. Lymphoid Leukemia L1210

Summary: Ascitic fluid from donor mouse is transferred into recipient BDF1 or CDF1 mice. Treatment begins 24 hours after implant. Results are expressed as a percentage of control survival time. Under normal conditions, the inoculum site for primary screening is i.p., the composition being tested is administered i.p., and the parameter is mean survival time. Origin of tumor line: induced in 1948 in spleen and lymph nodes of mice by painting skin with MCA. J Natl Cancer Inst. 13:1328, 1953.

| Animals | One sex used for all test and control animals in one experiment. |
|---|---|
| Tumor Transfer | Inject ip, 0.1 ml of diluted ascitic fluid containing $10^5$ cells |
| Propagation Time of Transfer | DBA/2 mice (or BDF1 or CDF1 for one generation). Day 6 or 7 |
| Testing Time of Transfer | $BDF_1$ (C57BU6 x DBA/2) or $CDF_1$ (BALB/c x DBA/2) Day 6 or 7 |
| Weight | Within a 3-g range, minimum weight of 18 g for males and 17 g for females. |
| Exp Size (n) | 6/group; No. of control groups varies according to number of test groups. |

Testing Schedule

| DAY | PROCEDURE |
|---|---|
| 0 | Implant tumor. Prepare materials. Run positive control in every odd-numbered experiment. Record survivors daily. |
| 1 | Weigh and randomize animals. Begin treatment with therapeutic composition. Typically, mice receive 1 $\mu$g of the test composition in 0.5 ml saline. Controls receive saline alone. Treatment is one dose/week. Any surviving mice are sacrificed after 4 wks of therapy. |
| 5 | Weigh animals and record. |
| 20 | If there are no survivors except those treated with positive control compound, evaluate |
| 30 | Kill all survivors and evaluate experiment. |

Quality Control: Acceptable control survival time is 8-10 days. Positive control compound is 5-fluorouracil; single dose is 200 mg/kg/injection, intermittent dose is 60 mg/kg/injection, and chronic dose is 20 mg/kg/injection. Ratio of tumor to control (T/C) lower limit for positive control compound is 135%.

Evaluation: Compute mean animal weight on Days 1 and 5, and at the completion of testing compute T/C for all test groups with > 65% survivors on Day 5. A T/C value 85% indicates a toxic test. An initial T/C 125% is considered necessary to demonstrate activity. A reproduced T/C 125% is considered worthy of further study. For confirmed activity a composition should have two multi-dose assays that produce a T/C 125%.

### B. Lymphocytic Leukemia P388

Summary: Ascitic fluid from donor mouse is implanted in recipient BDF1 or CDF1 mice. Treatment begins 24 hours after implant. Results are expressed as a percentage of control survival time. Under normal conditions, the inoculum site for primary screening is ip, the composition being tested is administered ip daily for 9 days, and the parameter is MedST. Origin of tumor line: induced in 1955 in a DBA/2 mouse by painting with MCA. Scientific

Proceedings, Pathologists and Bacteriologists33:603, 1957.

| Animals | One sex used for all test and control animals in one experiment. |
|---|---|
| Tumor Transfer | Inject ip, 0.1 ml of diluted ascitic fluid containing $10^6$ cells |
| Propagation Time of Transfer | DBA/2 mice (or BDF1 or CDF1 for one generation). Day 7 |
| Testing Time of Transfer | BDF$_1$ (C57BU6 x DBA/2) or CDF$_1$ (BALB/c x DBA/2) Day 6 or 7 |
| Weight | Within a 3-g range, minimum weight of 18 g for males and 17 g for females. |
| Exp Size (n) | 6/group; No. of control groups varies according to number of test groups. |

Testing Schedule

| DAY | PROCEDURE |
|---|---|
| 0 | Implant tumor. Prepare materials. Run positive control in every odd-numbered experiment. Record survivors daily. |
| 1 | Weigh and randomize animals. Begin treatment with therapeutic composition. Typically, mice receive 1 $\mu$g of the test composition in 0.5 ml saline. Controls receive saline alone. Treatment is one dose/week. Any surviving mice are sacrificed after 4 wks of therapy. |
| 5 | Weigh animals and record. |
| 20 | If there are no survivors except those treated with positive control compound, evaluate |
| 30 | Kill all survivors and evaluate experiment. |

Acceptable MedST is 9-14 days. Positive control compound is 5-fluorouracil: single dose is 200 mg/kg/injection, intermittent dose is 60 mg/kg/injection, and chronic dose is 20 mg/kg/injection. T/C lower limit for positive control compound is 135% Check control deaths, no takes, etc.

Quality Control: Acceptable MedST is 9-14 days. Positive control compound is 5-fluorouracil: single dose is 200 mg/kg/injection, intermittent dose is 60 mg/kg/injection, and chronic dose is 20 mg/kg/injection. T/C lower limit for positive control compound is 135%. Check control deaths, no takes, etc.

Evaluation: Compute mean animal weight on Days 1 and 5, and at the completion of testing compute T/C for all test groups with > 65% survivors on Day 5. A T/C value of 85% indicates a toxic test. An initial T/C of 125% is considered necessary to demonstrate activity. A reproduced T/C 125% is considered worthy of further study. For confirmed activity a composition should have two multi-dose assays that produce a T/C 125%.

## C. Melanotic Melanoma B16

Summary: Tumor homogenate is implanted ip or sc in BDF1 mice. Treatment begins 24 hours after either ip or sc implant or is delayed until an sc tumor of specified size (usually approximately 400 mg) can be palpated. Results expressed as a percentage of control survival time. The composition being tested is administered ip, and the parameter is mean survival time. Origin of tumor line: arose spontaneously in 1954 on the skin at the base of the ear in a C57BL/6 mouse. Handbook on Genetically Standardized Jax Mice. Jackson Memorial Laboratory, Bar Harbor, ME, 1962. See also Ann NY Acad Sci 100, Parts 1 and 2, 1963.

| Animals | One sex used for all test and control animals in one experiment. |
|---|---|
| Propagation Strain Tumor Transfer | C57BL/6 mice <br> Implant fragment sc by trochar or 12-g needle or tumor homogenate* every 10-14 days into axillary region with puncture in inguinal region. |
| Testing Strain Time of Transfer | $BDF_1$ (C57BU6 x DBA/2) <br> Excise sc tumor on Day 10-14 from donor mice and implant as above |
| Weight | Within a 3-g range, minimum weight of 18 g for males and 17 g for females. |
| Exp Size (n) | 10/group; No. of control groups varies according to number of test groups. |
| * Tumor homogenate: Mix 1 g or tumor with 10 ml of cold balanced salt solution, homogenize, and implant 0.5 ml of tumor homogenate ip or sc. Fragment: A 25-mg fragment may be implanted sc. | |

**Testing Schedule**

| DAY | PROCEDURE |
|---|---|
| 0 | Implant tumor. Prepare materials. Run positive control in every odd-numbered experiment. Record survivors daily./ |
| 1 | Weigh and randomize animals. Begin treatment with therapeutic composition. Typically, mice receive 1 $\mu$g of the test composition in 0.5 ml saline. Controls receive saline alone. Treatment is one dose/week. Any surviving mice are sacrificed after 8 wks of therapy. |
| 5 | Weigh animals and record. |
| 60 | Kill all survivors and evaluate experiment. |

Quality Control: Acceptable control survival time is 14-22 days. Positive control compound is 5-fluorouracil: single dose is 200 mg/kg/injection, intermittent dose is 60 mg/kg/injection, and chronic dose is 20 mg/kg/injection. T/C lower limit for positive control compound is 135% Check control deaths, no takes, etc.

Evaluation: Compute mean animal weight on Days 1 and 5, and at the completion of testing compute T/C for all test groups with > 65% survivors on Day 5. A T/C value of 85% indicates a toxic test. An initial T/C of 125% is considered necessary to demonstrate activity. A reproduced T/C 125% is considered worthy of further study. For confirmed activity a composition should have two multi-dose assays that produce a T/C 125%.

## Metastasis after IV Injection of Tumor Cells

$10^5$ B16 melanoma cells in 0.3 ml saline are injected intravenously in C57BL/6 mice. The mice are treated intravenously with 1g of the composition being tested in 0.5 ml saline. Controls receive saline alone. The treatment is given as one dose per week. Mice sacrificed after 4 weeks of therapy, the lungs are removed and metastases are enumerated.

## C. 3LL Lewis Lung Carcinoma

Summary: Tumor may be implanted sc as a 2-4 mm fragment, or im as a $2 \times 10^6$-cell inoculum. Treatment begins 24 hours after implant or is delayed until a tumor of specified size (usually approximately 400 mg) can be palpated. The composition being tested is administered ip daily for 11 days and the results are expressed as a percentage of the control. Origin of tumor line: arose spontaneously in 1951 as carcinoma of the lung in a C57BL/6 mouse. Cancer Res 15:39, 1955. See, also Malave, I. et al., J. Nat'l. Canc. Inst. 62:83-88 (1979).

| Animals | One sex used for all test and control animals in one experiment. |
|---|---|
| Propagation Strain Tumor Transfer | C57BU6 mice<br>Inject cells im in hind leg or implant fragment sc in axillary region with puncture in inguinal region. Transfer on day 12-14 |
| Testing Strain Time of Transfer | $BDF_1$ (C57BU6 x DBA/2) or C3H mice<br>Same as above |
| Weight Exp Size (n) | Within a 3-g range, minimum weight of 18 g for males and 17 g for females. 6/ group for sc implant, or 10/group for im implant.; No. of control groups varies according to number of test groups. |

### Testing Schedule

| DAY | PROCEDURE |
|---|---|
| 0 | Implant tumor. Prepare materials. Run positive control in every odd-numbered experiment. Record survivors daily. |
| 1 | Weigh and randomize animals. Begin treatment with therapeutic composition. Typically, mice receive 1 μg of the test composition in 0.5 ml saline. Controls receive saline alone. Treatment is one dose/week. Any surviving mice are sacrificed after 4 wks of therapy. |
| 5 | Weigh animals and record. |
| Final day | Kill all survivors and evaluate experiment. |

Quality Control: Acceptable im tumor weight on Day 12 is 500-2500 mg. Acceptable im tumor MedST is 18-28 days. Positive control compound is cyclophosphamide: 20 mg/kg/injection, qd, Days 1-11. Check control deaths, no takes, etc.

Evaluation: Compute mean animal weight when appropriate, and at the completion of testing compute T/C for all test groups. When the parameter is tumor weight, a reproducible T/C of 42% is considered necessary to demonstrate activity. When the parameter is survival time, a reproducible T/C of 125% is considered necessary to demonstrate activity. For confirmed activity a composition must have two multi-dose assays

## D. 3LL Lewis Lung Carcinoma Metastasis Model

This model has been utilized by a number of investigators. See, for example, Gorelik, E. et al., J. Nat'l. Canc. Inst. 65:1257-1264 (1980); Gorelik, E. et al., Rec. Results Canc. Res. 75:20-28 (1980); Isakov, N. et al., Invasion Metas. 2:12-32 (1982) Talmadge J.E. et al., J. Nat'l. Canc. Inst. 69:975-980 (1982); Hilgard, P. et al., Br. J. Cancer 35:78-86 (1977)).

Mice: male C57BL/6 mice, 2-3 months old. Tumor: The 3LL Lewis Lung Carcinoma was maintained by sc transfers in C57BL/6 mice. Following sc, im or intra-footpad transplantation, this tumor produces metastases, preferentially in the lungs. Single-cell suspensions are prepared from solid tumors by treating minced tumor tissue with a solution of 0.3% trypsin. Cells are washed 3 times with PBS (pH 7.4) and suspended in PBS. Viability of the 3LL cells prepared in this way is generally about 95-99% (by trypan blue dye exclusion). Viable tumor cells ($3 \times 10^4$ - $5 \times 10^6$) suspended in 0.05 ml PBS are injected into the right hind foot pads of C57BL/6 mice. The day of tumor appearance and the diameters of established tumors are measured by caliper every two days. Typically, mice receive 1 μg of the composition being tested in 0.5 ml saline. Controls receive saline alone. The treatment is given as one or two doses per week.

In experiments involving tumor excision, mice with tumors 8-10 mm in diameter are divided into two groups. In one group, legs with tumors are amputated after ligation above the knee joints. Mice in the second group are left intact as nonamputated tumor-bearing controls. Amputation of a tumor-free leg in a tumor-bearing mouse has no known effect on subsequent metastasis, ruling out possible effects of anesthesia, stress or surgery. Surgery is performed under Nembutal anesthesia (60 mg veterinary Nembutal per kg body weight).

## Determination of Metastasis Spread and Growth

Mice are killed 10-14 days after amputation. Lungs are removed and weighed. Lungs are fixed in Bouin's solution and the number of visible metastases is recorded. The diameters of the metastases are also measured using a binocular stereoscope equipped with a micrometer- containing ocular under 8X magnification. On the basis of the recorded diameters, it is possible to calculate the volume of each metastasis. To determine the total volume of metastases per lung, the mean number of visible metastases is multiplied by the mean volume of metastases. To further determine metastatic growth, it is possible to measure incorporation of 125IdUrd into lung cells (Thakur, M.L. et al., J. Lab. Clin. Med. 89:217-228 (1977). Ten days following tumor amputation, 25 mg of FdUrd is inoculated into the peritoneums of tumor-bearing (and, if used, tumor-resected mice. After 30 min, mice are given 1 mCi of 125IdUrd. One day later, lungs and spleens are removed and weighed, and a degree of 125IdUrd incorporation is measured using a gamma counter.

Statistics: Values representing the incidence of metastases and their growth in the lungs of tumor-bearing mice are not normally distributed. Therefore, non-parametric statistics such as the Mann-Whitney U-Test may be used for analysis. Study of this model by Gorelik *et al*. (1980, *supra*) showed that the size of the tumor cell inoculum determined the extent of metastatic growth. The rate of metastasis in the lungs of operated mice was different from primary tumor-bearing mice. Thus in the lungs of mice in which the primary tumor had been induced by inoculation of large doses of 3LL cells ($1-5 \times 10^6$) followed by surgical removal, the number of metastases was lower than that in nonoperated tumor-bearing mice, though the volume of metastases was higher than in the nonoperated controls. Using $^{125}$IdUrd incorporation as a measure of lung metastasis, no significant differences were found between the lungs of tumor- excised mice and tumor-bearing mice originally inoculated with $10^6$ 3LL cells. Amputation of tumors produced following inoculation of $10^5$ tumor cells dramatically accelerated metastatic growth. These results were in accord with the survival of mice after excision of local tumors. The phenomenon of acceleration of metastatic growth following excision of local tumors had been observed by other investigators. The growth rate and incidence of pulmonary metastasis were highest in mice inoculated with the lowest doses ($3 \times 10^4 - 10^5$ of tumor cells) and characterized also by the longest latency periods before local tumor appearance. Immunosuppression accelerated metastatic growth, though nonimmunologic mechanisms participate in the control exerted by the local tumor on lung metastasis development. These observations have implications for the prognosis of patients who undergo cancer surgery.

## E. A20 lymphoma

$10^6$ murine A20 lymphoma cells in 0.3 ml saline are injected subcutaneously in Balb/c mice. The mice are treated intravenously with 1g of the composition being tested in 0.5 ml saline. Controls receive saline alone. The treatment is given as one dose per week. Tumor growth is monitored daily by physical measurement of tumor size and calculation of total tumor volume. After 4 weeks of therapy the mice are sacrificed.

## HUMAN TUMOR XENOGRAFT MODELS

The preclinical discovery and development of anticancer drugs as implemented by the National Cancer Institute (NCI) consists of a series of test procedures, data review, and decision steps (Grever, MR, Semin Oncol., 19:622-638 (1992)). Test procedures are designed to provide comparative quantitative data, which in turn, permit selection of the best candidate agents from a given chemical or biological class. Below, we describe human tumor xenograft systems, emphasizing melanomas, that are currently employed in preclinical drug development.

Since 1975, the NCI approach to drug discovery involved prescreening of compounds in the i.p.-implanted murine P388 leukemia model (see above), followed by evaluation of selected compounds in a panel of transplantable tumors (Venditti, J.M. et al., In: Garrattini S et al., eds., Adv. Pharmacol and Chemother 2:1-20 (1984)) including human solid tumors. The latter was made possible through the development of immunodeficient athymic nude (*nu*/*nu*) mice and the transplantation into these mice of human tumor xenografts (Rygaard, J. et al., Acta Pathol. Microbiol. Scand. 77: 758-760 (1969); Giovanella, G.C. et al., J. Natl Canc. Inst. 51:615-619 (1973)). Studies assessing the metastatic potential of selected murine and human tumor-cell lines (B16, A-375, LOX-IMVI melanomas, and PC-3 prostate adenocarcinoma) and their suitability for experimental drug evaluation supported the importance of *in vivo* models derived from the implantation of tumor material in anatomically appropriate host tissues; such models are well suited for detailed evaluation of compounds that inhibit activity against specific tumor types. Beginning about 1990, the NCI began employing human tumor cell lines for large-scale drug screening ((Boyd, MR, In: DeVita, VT et al., Cancer: Principles and Practice of Oncology, Updates, vol 3, Philadelphia, Lippincott, 1989, pp 1-12; B. Teicher, ed., Anticancer Drug Development Guide: Preclinical Screening, Clinical Trials and Approval chapter 2). Cell lines derived from seven cancer types (brain, colon, leukemia, lung, melanoma, ovarian, and renal) were acquired from a wide range of sources, frozen, and subjected to a battery of *in vitro* and *in vivo* characterization.

This approach shifted the screening strategy from "compound-oriented" to "disease-oriented" drug discovery (Boyd, *supra*). Compounds of identified by the screen, demonstrating disease-specific, differential cytotoxicity such as the anti-melanoma activity of the compounds described herein, were considered "leads" for further preclinical evaluation. A battery of human tumor xenograft models was created to deal with such needs.

The approach used to establish s.c. xenografts from human tumor cell culture lines is that obtained from the NCI tumor repository at Frederick, Maryland). The cryopreserved cell lines are thawed, cultured in RPMI 1640 medium supplemented with 10%-heat-inactivated fetal bovine serum, and expanded until the population is sufficient to yield $\geq 10^8$ cells. Cells are harvested and then implanted s.c. into the axillary region of 10 athymic *nu/nu* mice ($10^7$ cells/0.5 ml/ mouse). Preferred housing conditions for these mice are as follows: mice are housed in sterile, polycarbonate, filter-capped microisolator cages (*e.g.*, from Lab. Products, Inc.), maintained in a barrier facility on 12-h light/dark cycles, and provided with sterilized food and water ad libitum. The implanted animals are observed twice weekly for tumor appearance. Growth of the solid tumors is monitored using *in situ* caliper measurements to determine tumor mass. Weights (mg) are calculated from measurements (mm) of two perpendicular dimensions (length and width) using the formula for a prolate ellipsoid and assuming a specific gravity of 1.0 g/cm$^3$ (Geran *et al., supra*). Fragments of these tumors may be subjected to histological, cytochemical, and ultrastructural analysis to monitor the characteristics of the *in vivo* material and to compare them with those of the *in vitro* lines and, where possible, with those reported for initial patient tumors (Stinson SF et al., Anticancer Res 12:1035-1054 (1992)). Both *in vitro* and *in vivo* tumor materials should exhibit characteristics consistent with tissue type and tumor of origin, though differences in the degree of differentiation between some of the cultured cell lines and corresponding xenograft materials are not uncommon.

The initial solid tumors established in mice are maintained by serial passage of 30-40 mg tumor fragments implanted s.c. near the axilla. Xenografts are generally not utilized for drug evaluation until the volume-doubling time has stabilized, usually around the fourth or fifth passage. The doubling time of xenografts derived from melanoma cell lines constituting both the initial (1990) and the modified (1993) human tumor cell line screens, are presented in Table 1 below. Also provided in the table is information on the take-rate of the tumors, and the experience of the NCI in the use of the tumors as early stage s.c. models. The doubling times were determined from vehicle-treated control mice used in drug evaluation experiments (data for passage numbers 4-20 are included). The doubling time is the median of the time interval for individual tumors to increase in size from 200-400 mg (usually a period of exponential growth). Both ranges and mean values are provided. Mean doubling times range from < 2 d for some tumors to > 10 d.

The *in vivo* growth characteristics of the xenografts determine their suitability for use in the evaluation of test agent antitumor activity, particularly when the xenografts are utilized as early stage s.c. models. As used herein, an early stage s.c. model is defined as one in which tumors are staged to 63-200 mg prior to the initiation of treatment. Growth characteristics considered in rating tumors include take-rate, time to reach 200 mg, doubling time, and susceptibility to spontaneous regression. As can be noted, the faster-growing tumors tend to receive the higher ratings.

## *Advanced-Stage Subcutaneous Xenograft Models*

Such s.c.-implanted tumor xenograft models are used to evaluate the antitumor activity of test agents under conditions that permit determination of clinically relevant parameters of activity, such as partial and complete regression and duration of remission (Martin DS et al., Cancer Treat Rep 68:37-38 (1984); Martin DS et al., Cancer Res. 46: 2189-2192 (1986); Stolfi, RL et al., J. Natl Canc Inst 80:52-55 (1988)). Tumor growth is monitored and test agent treatment is initiated when tumors reach a weight range of 100-400 mg (staging day, median weights approx. 200 mg), although depending on the xenograft, tumors may be staged at larger sizes. Tumor sizes and body weights are obtained approximately 2 times/wk. Through software programs (developed by staff of the Information Technology Branch of DTP of the NCI), data are stored, various parameters of effects are calculated, and data are presented in both graphic and tabular formats. Parameters of toxicity and antitumor activity are defined as follows:

1. **Toxicity**: Both drug-related deaths (DRD) and maximum percent relative mean net body weight losses are determined. A treated animal's death is presumed to be treatment-related if the animal dies within 15 d of the last treatment, and either its tumor weight is less than the lethal burden in control mice, or its net body weight loss at death is 20% greater than the mean net weight change of the controls at death or sacrifice. A DRD also may be designated by the investigator. The mean net body weight of each group of mice on each observation day is compared to the mean net body weight on staging day. Any weight loss that occurs is calculated as a percent of the staging day weight. These calculations also are made for the control mice, since tumor growth of some xenografts has an adverse effect on body weight.

2. **Optimal % T/C:** Changes in tumor weight (A weights) for each treated (T) and control (C) group are calculated for each day tumors are measured by subtracting the median tumor weight on the day of first treatment (staging day) from the median tumor weight on the specified observation day. These values are used to calculate a percent T/C as follows:

$$\% \ T/C \ = \ (\Delta T/\Delta C) \times 100 \text{ where } \Delta T > 0 \text{ or}$$
$$= \ (\Delta T/T_I) \times 100 \text{ where } \Delta T < 0 \qquad (1)$$

and $T_I$ is the median tumor weight at the start of treatment. The optimum (minimum) value obtained after the end of the first course of treatment is used to quantitate antitumor activity.

3. **Tumor growth delay**: This is expressed as a percentage by which the treated group weight is delayed in attaining a specified number of doublings; (from its staging day weight) compared to controls using the formula:

$$[(T - C)/C] \times 100 \qquad (2)$$

where T and C are the median times (in days) for treated and control groups, respectively, to attain the specified size (excluding tumor-free mice and DRDs). The growth delay is expressed as percentage of control to take into account the growth rate of the tumor since a growth delay based on (T - C) alone varies in significance with differences in tumor growth rates.

4. **Net log cell kill:** An estimate of the number of $\log_{10}$ units of cells killed at the end of treatment is calculated as:

$$\{[(T - C) - \text{duration of treatment}] \times 0.301 \ / \ \text{median doubling time}\} \qquad (3)$$

where the "doubling time" is the time required for tumors to increase in size from 200 to 400 mg, 0.301 is the $\log_{10}$ of 2, and T and C are the median times (in days) for treated and control tumors to achieve the specified number of doublings. If the duration of treatment is 0, then it can be seen from the formulae for net log cell kill and percent growth delay that log cell kill is proportional to percent growth delay. A log cell kill of 0 indicates that the cell population at the end of treatment is the same as it was at the start of treatment. A log cell kill of +6 indicates a 99.9999% reduction in the cell population.

5. **Tumor regression**: The importance of tumor regression in animal models as an end point of clinical relevance has been propounded by several investigators (Martin *et al.*, 1984, 1986 *supra*; Stolfi *et al., supra*). Regressions are defined-as partial if the tumor weight decreases to 50% or less of the, tumor weight at the start of treatment without dropping below 63 mg ($5 \times 5$ mm tumor). Both complete regressions (CRs) and tumor free survivors are defined by instances in which the tumor burden falls below measurable limits (<63 mg) during the experimental period. The two parameters differ by the observation of either tumor regrowth (in CR animals) or no regrowth (=tumor-free) prior to the final observation day. Although one can measure smaller tumors, the accuracy of measuring a s.c. tumor smaller than $4 \times 4$ mm or $5 \times 5$ mm (32 and 63 mg, respectively) is questionable. Also, once a relatively large tumor has regressed to 63 mg, the composition of the remaining mass may be only fibrous material/scar tissue. Measurement of tumor regrowth following cessation of treatment provides a more reliable indication of whether or not tumor cells survived treatment.

Most xenografts that grow s.c. may be used in an advanced-stage model, although for some tumors, the duration of the study may be limited by tumor necrosis. As mentioned previously, this model enables the measurement of clinically relevant parameters and provides a wealth of data on the effects of the test agent on tumor growth. Also, by staging day, the investigator is ensured that angiogenesis has occurred in the area of the tumor, and staging enables "no-takes" to be eliminated from the experiment. However, the model can be costly in terms of time and mice. For slower-growing tumors, the passage time required before sufficient mice can be implanted with tumors may be at least ~4 wks, and an additional 2-3 wks may be required before the tumors can be staged. To stage tumors, more mice (as many as 50-100% more) than are needed for actual drug testing must be implanted.

### Early Treatment and Early Stage Subcutaneous Xenograft Models

These models are similar to the advanced-stage model, but, because treatment is initiated earlier in the development of the tumor, useful tumors are those with ≥ 90% take-rate (or < 10% spontaneous regression rate). The "early treatment model" is defined as one in which treatment is initiated before tumors are measurable, *i.e.*, <63 mg. The "early stage" model as one in which treatment is initiated when tumor size ranges from 63-200 mg. The 63-mg size is used because it indicates that the original implant, about 30 mg, has demonstrated some growth. Parameters of toxicity are the same as those for the advanced-stage model; parameters of antitumor activity are similar. %T/C values are calculated directly from the median tumor weights on each observation day instead of being measured as changes (Δ) in tumor weights, and growth delays are based on the days after implant required for the tumors to reach a specified size, *e.g.*, 500 or 1000 mg. Tumor-free mice are recorded, but may be designated as "no-takes" or spontaneous regressions if the vehicle-treated control group contains >10% mice with similar growth characteristics. A "no-take" is a tumor that fails to become established and grow progressively.

A spontaneous regression (graft failure) is a tumor that, after a period of growth, decreases to ≤ 50% of its maximum size. Tumor regressions are not normally recorded, since they are not always a good indicator of antineoplastic effects in the early stage model. A major advantage of the early treatment model is the ability to use all implanted mice, which is why a good tumor take-rate is required. In practice, the tumors most suitable for this model tend to be the faster-growing ones.

### Challenge Survival Models

In another approach, the effect of human tumor growth on the lifespan of the host is determined. The LOX-IMVI melanoma has been used in this model. All mice dying or sacrificed owing to a moribund state or extensive ascites prior to the final observation day are used to calculate median day of death for treated (T) and control (C) groups. These values are then used to calculate a percent increase in life span ("ILS") as follows:

$$\% \text{ ILS} = [(T - C/C] \times 100 \qquad (4)$$

Where possible, titration groups are included to establish a tumor doubling time for use in $\log_{10}$ cell kill calculations. A death (or sacrifice) may be designated as drug-related based on visual observations and/or the results of necropsy. Otherwise, treated animal deaths are- designated as treatment-related if the day of death precedes the mean day of death of the controls (-2SD) or if the animal dies without evidence of tumor within 15 days of the last treatment.

### Response of Xenograft Models to Standard Agents

In obtaining drug sensitivity profiles for the advanced-stage s.c. xenograft models, the test agent is evaluated following i.p. administration at multiple dose levels. The activity ratings are based on the optimal effects attained with the maximally tolerated dose (<$LD_{20}$) of each drug for a given treatment schedule which is selected on the basis of the doubling time of a given tumor, with longer intervals between treatments for slower growing tumors.

### Strategy for Initial Compound Evaluation In Vivo

The *in vitro* primary screens provide a basis for selecting the most appropriate tumor lines to use for follow-up *in vivo* testing, with each compound and combination of agents. As described herein tested only against xenografts derived from cell lines demonstrating the greatest sensitivity to the agent *in vitro.* The early strategy for *in vivo* testing emphasized the treatment of animals bearing advanced-stage tumors.

Unless specific information is available to guide dose selection, single mice are preferably treated with single ip bolus doses of 100, 200, and 400 mg/kg and observed for 14 d. Sequential 3-dose studies may be conducted as necessary until a nonlethal dose range is established. The test agent is then evaluated preferably in three s.c. xenograft models using tumors that are among the most sensitive to the test agent *in vitro* and that are suitable for use as early stage models. The compounds are administered ip, as suspensions if necessary, on schedules based, with some exceptions, on the mass doubling time of the tumor. For example, for doubling times of 1.3-2.5, 2.6-5.9, and 6-10 d, preferred schedules are: daily for five treatments (qd x 5), every fourth day for three treatments (q4d x 3), and every seventh day for three treatments (q7d x 3). For most tumors, the interval between individual treatments approximates the doubling time of the tumors, and the treatment period allows a 0.5-1.0 $\log_{10}$ unit of control tumor growth. For tumors staged at 100-200 mg, the tumor sizes of the controls at the end of treatment should range from 500-2000 mg, which allows sufficient time after treatment to evaluate the effects of the test agent before it becomes necessary to sacrifice mice owing to tumor size.

### *Detailed Drug Studies*

Once a compound has been identified as demonstrating *in vivo* efficacy in initial evaluations, more detailed studies are designed and conducted in human tumor xenograft models to explore further the compound's therapeutic potential. By varying the concentration and exposure time of the tumor cells and the host to the drug, it is possible to devise and recommend treatment strategies designed to optimize antitumor activity.

The importance of "concentration $\times$ time" on the antitumor effects of test agents were well illustrated by data obtained with amino-20M-camptothecin (Plowman, J. *et al.*, 1997, *supra*). Those results indicated that maintaining the plasma concentration above a threshold level for a prolonged period of time was required for optimal therapeutic effects.

### *Hollow-Fiber Assays: A Newer Approach to In Vivo Drug Testing*

This model uses human tumor cell lines growing in hollow fibers and is intended as a prioritization tool through which lead compounds identified in an *in vitro* screen would pass. In brief, tumor cells are inoculated into hollow fibers (1 mm internal diameter), and the fibers are heat-sealed and cut at 2-cm intervals. These samples are maintained for 24-48 h *in vitro* and then implanted into nude mice. At the time of implantation, a representative set of fibers is assayed for viable cell mass by the "stable end point" MTT dye conversion technique (Alley, MC et al., Canc Res 51:1247-1256 (1991)) in order to determine the "time zero" cell mass for each cell line. The mice are treated with test agents on a daily treatment schedule, and the fibers are collected 6-8 d postimplantation. At collection, the quantity of viable cells contained in the fibers is measured. The antitumor effects of the test agents are determined from the changes in viable cell mass in the fibers collected from compound-treated and diluent-treated mice. Using this technique, three different tumor cell lines can be grown conveniently in each of two physiologic sites (*e.g.*, ip and sc) within each experimental mouse. Thus, this model provides a method for administering a test agent ip to evaluate its effect against tumor cells growing in both the ip cavity and the s.c. compartment. Such simultaneous assessment of multiple tumor cell lines grown in two physiologic compartments should permit rapid identification of lead compounds with the greatest promise of clinical effectiveness.

This *in vivo/ in vitro* hollow-fiber system may be well suited for the prioritization of compounds for more advanced stages of *in vivo* drug evaluation. Practically, this system can be viewed as a means to facilitate traditional chemotherapeutic testing, since it is rapid, sensitive, and is broadly applicable to a variety of human tumor cell types. Additionally, it requires only a limited quantity of test compound, a relatively small number of animals and, therefore, limited animal housing space.

## Xenograft Model of Metastasis

The compounds of this invention are also tested for inhibition of late metastasis using an experimental metastasis model such as that described by Crowley, C.W. et al., Proc. Natl. Acad. Sci. USA 90 5021-5025 (1993)). Late metastasis involves the steps of attachment and extravasation of tumor cells, local invasion, seeding, proliferation and angiogenesis. Human melanoma cells transfected with a reporter gene, preferably the green fluorescent protein (GFP) gene, but as an alternative with a gene encoding the enzymes chloramphenicol acetyl-transferase (CAT), luciferase or LacZ, are inoculated into nude mice. This permits utilization of either of these markers (fluorescence detection of GFP or histochemical colorimetric detection of enzymatic activity) to follow the fate of these cells. Cells are injected, preferably iv, and metastases identified after about 14 days, particularly in the lungs but also in regional lymph nodes, femurs and brain. This mimics the organ tropism of naturally occurring metastases in human melanoma. For example, GFP-expressing melanoma cells ($10^6$ cells per mouse) are injected i.v. into the tail veins of nude mice. Animals are treated with a test composition at 100$\mu$g/animal/day given q.d. IP. Single metastatic cells and foci are visualized and quantitated by fluorescence microscopy or light microscopic histochemistry or by grinding the tissue and quantitative colorimetric assay of the detectable label.

### Human Melanoma/SCID Mouse Model

Safrians, S. et al., Int'l J. Canc. 66:131-1f58 (1996), incorporated by reference) described studies in a human melanoma/SCID mouse model. The highly metastatic human melanoma line. C8161 (Welch *et al., 1991)* was transfected with antibiotic-selectable markers (with the vectors *pSV2neo* and *pSV2hygro*) using conventional methods. As clones emerged when the cells were grown in medium containing G-418 and hygromycin, the concentrations of the two agents were reduced respectively to 0.2 mg/ml and 0.1 mg/ml. Emerging clones were identified within 3-4 weeks and removed with cloning rings. Ploidy studies and karyotype analyses were performed to verify that selected clones bearing either of the two markers had no gross alterations in DNA content nor had they undergone changes in doubling time, tumorigenicity, constitutive levels of secreted collagenases, *in vitro*, Matrigel invasion or, most importantly, metastatic phenotype. Both *neo⁻* C8161 and *hyg⁻* C8161, like the parental line, demonstrate strong cytoplasmic immunoreactivity of cytokeratins 8 and 18, which facilitates their detection within the organs.

Between 5 x 10⁴ and 5 x 10⁶ *neo⁻* and/or *hyg⁻* C8161 cells suspended in 0.2 ml Hanks' balanced salt solution (HBSS) are injected either s.c. in a right dorsolateral flank region (assay for spontaneous metastasis) or i.v. in the tail vein (hematogenous0 metastasis) or via both routes at successive intervals. Animals are killed at various intervals (preferably ranging from 2 to 8 weeks), the organs are removed and metastatic colonies are quantified to determine the distribution of tumor cells from hematogenous dissemination. The size of the primary tumor as well as the number and distribution of metastases are determined.

Representative mice are subjected to histopathological and immunocytochemical studies to further document the presence of metastases throughout the major organs. Number and size (greatest diameter) of the colonies can be tabulated by digital image analysis, *e.g.*, as described by Fu, Y.S. et al., Anat. Quant. Cytol. Histol. 11:187-195 (1989)).

For determination of colonies, explants of lung, liver, spleen, para-aortic lymph nodes, kidney, adrenal glands and s.c. tissues are washed, minced into pieces of 1-2 mm³ and the pieces pulverized in a Tekman tissue pounder for 5 min. The pulverized contents are filtered through a sieve, incubated in a dissociation medium (MEM supplemented with 10% FCS, 200 U/ml of collagenase type I and 100 μg/ml of DNase type I) for 8 hr at 37°C with gentle agitation. Thereafter, the resulting cell suspension is washed and resuspended in regular medium (*e.g.*, MEM with 10% FCS supplemented with the selecting antibiotic (G-418 or hygromycin). The explants are fed as described by Safrians *et al., supra*, and the number of clonal outgrowths of tumor cells is determined after fixation with ethanol and staining with a monoclonal antibody to cytokeratins 8 and 18. The number of colonies is counted over an 80-cm² area. If desired, a parallel set of experiments can be conducted wherein clonal outgrowths are not fixed and stained but rather are retrieved fresh with cloning rings and pooled after only a few divisions for other measurements such as secretion of collagenases (by substrate gel electrophoresis) and Matrigel invasion.

Modified Matrigel invasion assays are performed as described by others (Hendrix, M.J.C. et al., Cancer Lett., 38:137-147 (1987); Albini, A. et al., Cancer Res., 47 3239-3245 (1987); Melchiori, A., Cancer Res. 52:2353-2356 (1992)). Substrate gel electrophoresis of conditioned media from the aforementioned clones are analyzed as described by others (Herron, G.S. et al., J. Biol. Chem. 261:2814-2818 (1986); Ballin, M. et al., Biochem. Biophys. Res. Comm., 154:832-838 (1988)).

All experiments are performed with groups that preferably have 10 mice. Results are analyzed with standard statistical tests. C8161 cells demonstrate significant numbers of both spontaneous and hematogenous metastasis. Significant numbers of hematogenous metastases may be produced almost exclusively in the lungs with an injection of 5 x 10⁵ cell (and larger numbers result in extrapulmonary metastases).

According to Safrians *et al., supra,* i.v. injections of 5 x 10⁵ tumor cells 1 week after an s.c. flank injection of an equal number of tumor cells followed by an additional 5-week interval yielded a ratio of 2:1 hematogenous:spontaneous pulmonary metastases and an overall pulmonary tumor burden of 1.25 g (over a normal pulmonary weight: 0.2 g). With this regimen, numerous extrapulmonary metastatic clones could be retrieved from spleen, liver, kidneys, adrenal gland, para-aortic lymph nodes and s.c. sites. The vast majority of these clones represent spontaneous metastases from the locally growing tumor. Similar results were obtained with C8161 carrying either of the antibiotic resistance markers discussed above.

Having now generally described the invention, the same will be more readily understood through reference to the following examples which are provided by way of illustration, and are not intended to be limiting of the present invention, unless specified.

## EXAMPLE I

### VEGF Induction and TSP-1 Inhibition in Tumor Cells

From gene expression analysis studies performed on a human leiomyosarcoma cell line (SK-LMS-1) (data not shown), the present inventors found that VEGF expression increased after HGF/SF treatment as previously reported for other tumor cells lines (13, 14). The present inventors performed Northern analysis on SK-LMS-1 cells and showed that HGF/SF treatment induced VEGF and expression persisted for as long as it was measured, up to 48 hours (Fig. 1A). VEGF was also elevated in long term cultures of SK-LMS-1 cells autocrine for HGF/SF (SK/HGF, 15) (Fig. 1A). The present inventors also examined MDA-MB-231 cells, a human breast cancer cell line, (Fig. 1B) and, as with SK-LMS-1, after HGF/SF treatment, the levels of VEGF increased and persisted for 48 hours. In gene expression studies, the present inventors also observed that the anti-angiogenic factor, TSP-1, decreased in response to HGF/SF stimulation and the present inventors observed a significant decrease in TSP-1 expression in SK-LMS-1 cells by Northern Blot analyses (Fig. 1A) following HGF/SF treatment. This effect was seen as early as 6 hours after HGF treatment and continued to 48 hours. More dramatically, TSP-1 expression was eliminated in the SK/HGF cell line. The down-regulation of TSP-1 by HGF/SF was also observed in MDA-MB-231 cells at 24 and 48 hours after HGF treatment (Fig. 1B).

## EXAMPLE II

### MAP Kinase Inhibitors Block VEGF Induction and TSP-1 Down-Regulation

HGF/SF, acting through its tyrosine kinase receptor, Met, is known to activate several intracellular signaling pathways, including MAP kinase, PI3 kinase and Stat3 (1, 16). The present inventors asked which pathways might be involved in regulating VEGF and TSP-1 expression. The present inventors treated SK-LMS-1 and MDA-MB-231 cells with (or without) various inhibitors for one hour, followed by HGF/SF stimulation for 15 minutes (Figs. 2A/1 and 2A/2). Met receptor is tyrosine-phosphorylated in response to HGF/SF, followed by the activation of downstream targets of Erk (p44/42 MAPK) and Akt/PI3 kinase. MAP kinase specific inhibitors PD98059 or U0126 blocked the activation of Erk, while the PI3 kinase specific inhibitor LY294002 blocked Akt activation (Fig. 2A/1-2A/2). RNA samples from SK-LMS-1 and MDA-MB-231 cells treated with individual inhibitors followed by HGF/SF treatment for 24 hours were analyzed by Northern Blot analyses. HGF/SF-induced shut off of TSP-1 was blocked by PD98059 or U0126 but was not affected by LY294002 (Figs. 2B/1-2B/2), nor by overexpression of Stat3β, a dominant-negative form of Stat3 (Fig. 2C) (17). These results indicated that neither Akt nor Stat3 influenced TSP-1 down-regulation by HGF/SF. In MDA-MB-231 cells, the present inventors observed a dramatic effect from the MEK MAP kinase inhibitors on TSP-1 (Fig. 2B/2). Interestingly, after PD98059 or U0126 treatment, TSP-1 expression was higher than the basal level in MDA-MB-231 cells (Fig. 2B/2).
This finding is consistent with MDA-MB-231 cells having a high constitutive level of MAP kinase activity which is inhibited by PD98059 and U0126 (Fig. 2A/2). Thus, HGF/SF mediated down-regulation of TSP-1 is dependent on the MAP kinase pathway and is independent of PI3 kinase and Stat3 pathways.
By contrast to TSP-1, the present inventors found that PD98059 and U0126 suppressed the expression of VEGF induced by HGF/SF in MDA-MB-231 cells, but only slightly in SK-LMS-1 cells (Fig. 2B/1). Moreover, VEGF expression was also suppressed by LY294002 and Stat3β (Figs. 2B/1, 2B/2 & 2C). These data are consistent with previous reports showing that MAP kinase, PI3 kinase and Stat3 pathways positively regulate VEGF expression (13, 18). These results indicated that HGF/SF-induced down-regulation of TSP-1 and up-regulation of VEGF is differentially mediated by distinct intracellular pathways.
Consistently, when MDA-MB-231 cells were treated with PD98059 or U0126 in the absence of HGF/SF induction, a similar dual regulation was observed as down-regulation of VEGF and up-regulation of TSP-1 (Fig. 2D). More importantly, a dramatic down-regulation of VEGF was observed along with a comparable up-regulation of TSP-1 expression when treating MDA-MB-231 cells with Lethal factor (LF), another known MAPK inhibitor (Fig. 2D). This report is the first to show that LF can dually regulate angiogenic effectors by increasing TSP-1 expression and decreasing VEGF expression simultaneously, and thereby inhibit angiogenesis. These results indicate that MAPK inhibitors such as LF are promising therapeutic reagents for inhibiting angiogenesis of both HGF/SF-dependent and independent tumors.

## EXAMPLE III

### TSP-1 Overexpression Inhibits Tumor Cell Growth via anti Angiogenic Effects

The next study tested whether down-regulation of TSP-1 by HGF/SF had any biological effect on HGF/SF-induced tumor growth. TSP-1 was overexpressed in SK/HGF cells to generate SK/HGF-TSP1 cells (Fig. 3A). Overexpression of TSP-1 has no effect on cell proliferation or anchorage-independent growth compared the parental SK/HGF cells *in vitro* (Figs. 5A-5B). To test whether TSP-1 influences tumorigenicity, the present inventors SK/HGF and SK/HGF-TSP1 cells were subcutaneously implanted in *athymic nude* mice, and their tumor growth rates were compared. At early times, no growth differences were observed between the SK/HGF and SK/HGF-TSP1 groups. However, when the tumors grew to a certain size, differences between became more apparent (Student's *t test* p<0.025). HGF/SF-dependent tumor growth was partially inhibited by TSP-1 overexpression (Figs. 3B 3C). TSP-1 protein expression was confirmed in the SK/HGF-TSP1 tumor group by Western blot analysis (Fig. 3D). These results indicated that down-regulation of TSP-1 by HGF/SF contributes to tumor development.
To test whether the inhibition of tumor growth by TSP-1 was due to an extrinsic effect by preventing tumor angiogenesis, the present inventors performed immunohistochemical staining using antibodies against mouse endothelial cell surface marker CD31 to detect the number of blood vessels in SK/HGF and SK/HGF-TSP-1 tumor sections. The average number of CD31-positive vessels in SK/HGF control tumor group was significantly higher than that in SK/HGF-TSP1 tumor group [Fig. 4A and Figs. 4B/1-6 (Student's *t test* p<0.01)]. These results indicated that TSP-1 inhibition of HGF/SF-induced tumor growth is mediated at least in part through suppression of tumor angiogenesis.

**DISCUSSION OF EXAMPLES**

The foregoing results provide insight into the mechanism of how HGF induces tumor angiogenesis as follows. See (Fig. 6): (i) HGF/SF itself acts directly on endothelial cells, inducing proliferation and migration *in vitro* (10-12); (ii) HGF/SF up-regulates the expression of a pro-angiogenic factor such as VEGF (Fig. 1A-1C) (13, 14) and VEGF activates endothelial cells to proliferate and migrate (5); and (iii) HGF/SF signaling down-regulates the expression of TSP-1, an angiogenesis antagonist (Fig. 1A-1D). This regulation is systemic and qualifies as a dominant acting angiogenic switch (4) and would be expected to dramatically enhance neovascularization. Oncogenes such as Ras and Myc have also been shown to coordinate the expression of VEGF and TSP-1 (19, 20).

Given that angiogenic factors like HGF/SF can simultaneously up-regulate VEGF and down-regulate TSP-1 expression (Figs. 1A-1C), the combination of anti-VEGF neutralizing antibodies plus therapeutic TSP-1 are expected to synergize to inhibit tumor angiogenesis and tumor growth. An alternative strategy targets the signaling pathways that are responsible for inhibiting TSP-1 shut off and VEGF expression. Here it was demonstrated that the MAP kinase pathway played a dual role in regulating the expression of angiogenic effectors, but it is especially effective in preventing the negative regulation of TSP-1 expression induced by HGF/SF. It is less effective in controlling the up-regulation of VEGF expression in some tumor cells such as SK-LMS-1 (Fig. 2B/1). However, the MAP kinase pathway is an important and intrinsic target in many tumor types (23).

A combination of a small molecule MAP kinase inhibitor coupled with a neutralizing anti-VEGF therapy are predicted to be effective. It is noteworthy is that tumor lethal factor (TLF), the anthrax toxin is a potent MAP kinase inhibitor (24; Int'1 Patent Pub. WO 99/50439; U.S. Patent Public. 20030096333) and also dramatically suppresses tumor angiogenesis (25; Int'1 Patent Pub. WO 02/076496). The mechanisms underlying the inhibition of tumor angiogenesis by TLF is not clear, but according to the present invention, TLF can increase expression of the anti-angiogenic factor TSP1 from tumor cells, while decreasing the VEGF (Fig. 2D). Direct targeting of HGF/SF and its receptor, Met, could have potent intrinsic and extrinsic antitumor activity. Anti-HGF/SF neutralizing antibodies and the HGF/SF antagonist, HGF/NK4, not only inhibit angiogenesis but also inhibit cell proliferation and invasion. This combination was shown to effectively inhibit tumor growth in animal models (26, 27).

***Some of the Cited References (in parentheses above)***

1. L. Trusolino, P.M. Comoglio, Nat Rev Cancer 4, 289-300 (2002).
2. G. F. Vande Woude et al., in CIBA Found. Symp.: Plasminogen-Related Growth Factors, G. R. Bock, J. A. Goode, Eds. (Wiley, New York, 1997), vol. 212, 119-132.
3. D. Hanahan, R. A. Weinberg, Cell 100, 57-70 (2000).
4. D. Hanahan, J. Folkman, Cell 86, 353-364 (1996).
5. N. Ferrara, Semin. Oncol. 29, 10-14 (2002).
6. I. Sargiannidou, J. Zhou, G. P. Tuszynski, Exp. Biol. Med. 226, 726-733 (2001).
7. B. Jimenez et al., Nat. Med. 6, 41-48 (2000).
8. K. M. Dameron, O. V. Volpert, M. A. Tainsky, N. Bouck, Science 265, 1582-1584 (1994).
9. K. Bleuel et al., Proc. Natl. Acad. Sci. U S A 96, 2065-2070 (1999).
10. F. Bussolino et al., J. Cell Biol. 119, 629-641 (1992).
11. D. S. Grant et al., Proc. Natl. Acad. Sci. U S A 90, 1937-1941 (1993).
12. E. M. Rosen, I. D. Goldberg, Adv. Cancer. Res. 67, 257-279 (1995).
13. G. Dong et al., Cancer Res. 61, 5911-5918 (2001).
14. T. Moriyama et al., Biochem. Biophys. Res. Commun. 249, 73-77 (1998).
15. M. Jeffers, S. Rong, G. F. Vande Woude, Mol Cell Biol. 16, 1115-1125 (1996).
16. K. A. Furge, Y. W. Zhang, G. F. Vande Woude, Oncogene 19, 5582-5589 (2000).
17. Y. W. Zhang, L. M. Wang, R. Jove, G. F. Vande Woude, Oncogene 21, 217-226 (2002).
18. G. Niu et al., Oncogene 21, 2000-2008 (2002).
19. J. Rak et al., Cancer Res. 60, 490-498 (2000).
20. T. A. Baudino et al., Genes Dev. 16,2530-2543 (2002).
21. R. Kerbel, J. Folkman, Nat. Rev. Cancer 2, 727-739 (2002).
22. N. Ferrara, Nat. Rev. Cancer 2, 795-803 (2002).
23. T. S. Lewis, P. S. Shapiro, N. G. Ahn, Adv. Cancer Res. 74, 49-139 (1998).
24. N. S. Duesbery et al., Science 280, 734-737 (1998).
25. N. S. Duesbery et al., Proc. Natl. Acad. Sci. U S A 98, 4089-4094 (2001).
26. B. Cao et al., Proc. Natl. Acad. Sci. U S A 98, 7443-7448 (2001).

27. K. Kuba et al., Cancer Res. 60, 6737-6743 (2000).

Having now fully described this invention, it will be appreciated by those skilled in the art that the same can be performed within a wide range of equivalent parameters, concentrations, and conditions without departing from the spirit and scope of the invention and without undue experimentation.

**Claims**

1. Use of

    (a) an anti-angiogenic factor selected from TSP-1, a TSP-1 agonist or mimic; in combination with
    (b) a VEGF inhibitor;
    in the manufacture of a medicament for inhibiting tumor angiogenesis.

2. A use according to claim 1, wherein the factor or agonist of (a) and the inhibitor of (b)

    (i) inhibit endothelial cell proliferation,
    (ii) inhibit endothelial cell migration, and/or
    (iii) induce endothelial cell apoptosis
    thereby inhibiting said angiogenesis.

3. A use according to claim 1 or 2, wherein the VEGF inhibitor is a molecule that targets VEGF expression dependent signaling pathways.

4. A use according to claim 1 or 2, wherein the VEGF inhibitor inhibits VEGF expression.

5. A use according to claim 1 or 2, wherein the VEGF inhibitor is an anti-VEGF antibody.

6. A use according to claim 5, wherein the anti-VEGF antibody is a monoclonal antibody.

7. A use according to any one of the preceding claims, wherein the medicament is for inhibiting tumor angiogenesis in a subject *in vivo,* which subject is susceptible to, or at risk of, tumor growth or metastasis, or in which subject said tumor growth or metastasis is ongoing.

8. A composition comprising:

    (a) an anti-angiogenic factor selected from TSP-1, a TSP-1 agonist or mimic; in combination with
    (b) a VEGF inhibitor.

9. A composition according to claim 8, wherein the VEGF inhibitor is a molecule that targets VEGF expression dependent signaling pathways.

10. A composition according to claim 8, wherein the VEGF inhibitor inhibits VEGF expression.

11. A composition according to claim 8, wherein the VEGF inhibitor is an anti-VEGF antibody.

12. A composition according to claim 11, wherein the anti-VEGF antibody is a monoclonal antibody.

13. A composition according to any one of claims 8 - 12, further comprising a pharmaceutically acceptable carrier or excipient.

**Patentansprüche**

1. Verwendung von

    (a) einem anti-angiogenen Faktor, ausgewählt aus TSP-1, einem TSP-1-Agonisten oder Nachahmer (Mimic);

in Kombination mit
(b) einem VEGF-Inhibitor;
bei der Herstellung von einem Medikament zum Hemmen von Tumor-Angiogenese.

2. Verwendung nach Anspruch 1, wobei der Faktor oder Agonist von (a) und der Inhibitor von (b)

(i) endotheliale Zell-Proliferation hemmen,
(ii) endotheliale Zell-Migration hemmen, und/oder
(iii) endotheliale Zell-Apoptose induzieren, wodurch die Angiogenese gehemmt wird.

3. Verwendung nach Anspruch 1 oder 2, wobei der VEGF-Inhibitor ein Molekül ist, das auf VEGF-Expressions-abhängige Signal-Pathways abzielt.

4. Verwendung nach Anspruch 1 oder 2, wobei der VEGF-Inhibitor VEGF-Expression hemmt.

5. Verwendung nach Anspruch 1 oder 2, wobei der VEGF-Inhibitor ein Anti-VEGF-Antikörper ist.

6. Verwendung nach Anspruch 5, wobei der Anti-VEGF-Antikörper ein monoklonaler Antikörper ist.

7. Verwendung nach einem der vorangehenden Ansprüche, wobei das Medikament zum Hemmen von Tumor-Angiogenese bei einem Patienten *in vivo* vorgesehen ist, wobei der Patient anfällig ist oder ein Risiko aufweist für Tumor-Wachstum oder Metastase, oder bei dem Patienten das Tumor-Wachstum oder Metastase bereits entwickelt ist.

8. Zusammensetzung, umfassend:

(a) einen anti-angiogenen Faktor, ausgewählt aus TSP-1, einem TSP-1-Agonisten oder Nachahmer (Mimic); in Kombination mit
(b) einem VEGF-Inhibitor.

9. Zusammensetzung nach Anspruch 8, wobei der VEGF-Inhibitor ein Molekül ist, das auf VEGF-Expressions-abhängige Signal-Pathways abzielt.

10. Zusammensetzung nach Anspruch 8, wobei der VEGF-Inhibitor VEGF-Expression hemmt.

11. Zusammensetzung nach Anspruch 8, wobei der VEGF-Inhibitor ein Anti-VEGF-Antikörper ist.

12. Zusammensetzung nach Anspruch 11, wobei der Anti-VEGF-Antikörper ein monoklonaler Antikörper ist.

13. Zusammensetzung nach einem der Ansprüche 8 - 12, weiterhin umfassend einen pharmazeutisch verträglichen Träger oder Exzipienten.

**Revendications**

1. Utilisation de

(a) un facteur anti-angiogénique sélectionné parmi TSP-1, un agoniste ou un mimétique de TSP-1 ; en combinaison avec
(b) un inhibiteur du VEGF ; dans la fabrication d'un médicament destiné à inhiber l'angiogenèse tumorale.

2. Utilisation selon la revendication 1, où le facteur ou l'agoniste de (a) et l'inhibiteur de (b)

(i) inhibent la prolifération des cellules endothéliales,
(ii) inhibent la migration des cellules endothéliales, et/ou
(iii) induisent l'apoptose des cellules endothéliales ce qui inhibe ainsi ladite angiogenèse.

3. Utilisation selon la revendication 1 ou 2, où l'inhibiteur du VEGF est une molécule qui cible des voies de signalisation dépendantes de l'expression du VEGF.

4.  Utilisation selon la revendication 1 ou 2, où l'inhibiteur du VEGF inhibe l'expression du VEGF.

5.  Utilisation selon la revendication 1 ou 2, où l'inhibiteur du VEGF est un anticorps anti-VEGF.

6.  Utilisation selon la revendication 5, où l'anticorps anti-VEGF est un anticorps monoclonal.

7.  Utilisation selon l'une quelconque des revendications précédentes, où le médicament est destiné à inhiber l'angiogenèse tumorale chez un sujet *in vivo,* lequel sujet est prédisposé ou est exposé à un risque de croissance tumorale ou de métastase, ou ladite croissance tumorale ou métastase est en cours chez le sujet.

8.  Composition comprenant:

    (a) un facteur anti-angiogénique sélectionné parmi TSP-1, un agoniste ou un mimétique de TSP-1 ; en combinaison avec
    (b) un inhibiteur du VEGF.

9.  Composition selon la revendication 8, où l'inhibiteur du VEGF est une molécule qui cible des voies de signalisation dépendantes de l'expression du VEGF.

10. Composition selon la revendication 8, où l'inhibiteur du VEGF inhibe l'expression du VEGF.

11. Composition selon la revendication 8, où l'inhibiteur du VEGF est un anticorps anti-VEGF.

12. Composition selon la revendication 11, où l'anticorps anti-VEGF est un anticorps monoclonal.

13. Composition selon l'une quelconque des revendications 8 à 12, comprenant en outre un véhicule ou un excipient pharmaceutiquement acceptable.

Fig 1A

Fig. 1B

Fig 1C

Fig. 2A/2

Fig. 2A/1

Fig. 2C

Fig. 2B/2

Fig. 2B/1

## Fig. 2D

Fig. 3C

Fig. 3D

Fig. 3A

Fig. 3B

Fig. 4B/4

Fig. 4B/5

Fig. 4B/6

SK/HGF-TSP1

SK/HGF Cont.

Fig. 4B/1

Fig. 4B/2

Fig. 4B/3

Fig. 4A

Vessel Number/3 Fields (10x)

SK/HGF Cont.

SK/HGF-TSP1

120
100
80
60
40
20
0

**Fig. 5A**

**Fig. 5B**

EP 1 648 493 B1

Fig. 6

## REFERENCES CITED IN THE DESCRIPTION

### Patent documents cited in the description

- WO 0166144 A
- US 5591631 A
- US 5677274 A
- US 55677304 P
- US 60556473 B
- US 0305346 W
- US 0305028 W
- US 6506559 B
- US 20030206887 A
- WO 9907409 A
- WO 9932619 A
- WO 0001846 A
- WO 0044914 A
- WO 0044895 A
- WO 0129058 A
- WO 0136646 A
- WO 0175164 A
- WO 0192513 A
- WO 0189304 A
- WO 0190401 A
- WO 0216620 A
- WO 0229858 A
- US 556473 A
- WO 0134650 A1
- US 5686292 A
- US 6207152 B
- US 6214344 B, Schwall
- US 6099841 A, Hillan
- WO 9220792 A
- US 4376110 A
- US 4816567 A
- US 6331415 B
- US 121801 B
- WO 9404679 A
- EP 404097 A
- WO 9311161 A
- US 3969287 A
- US 3691016 A
- US 4195128 A
- US 4247642 A
- US 4229537 A
- US 4330440 A
- US 5364934 A
- WO 8705330 A
- US 4640835 A
- US 4496689 A
- US 4301144 A
- US 4670417 A
- US 4791192 A
- US 4179337 A
- WO 9300109 A
- WO 9950439 A
- US 20030096333 A
- WO 02076496 A

### Non-patent literature cited in the description

- **Allegrini et al.** *Proc. Ann. Meeting of the American Association for Cancer Res.,* 2000, vol. 41, 813
- **de Vos et al.** *Eur. J. Cancer,* 2002, vol. 38, 878-879
- **Armstrong et al.** *Matrix Biol.,* 2003, vol. 22, 63-71
- **Guo et al.** *J. Peptide Res.,* 1997, vol. 50, 210-221
- **Streit et al.** *American J. Pathol.,* 1999, vol. 155, 441-452
- **Reiher et al.** *Int. J. Cancer,* vol. 98, 682-689
- **Feige.** *Pathologie Biologie,* vol. 47, 339-344
- **Lawler.** *J. Cell. Mol. Medicin,* 2002, vol. 6, 1582-1838
- **Henkin et al.** *Proc. Ann. Meeting of the American Association for Cancer Res.,* 2002, vol. 43, 180
- **Vailé et al.** *Curr. Pharm. Design,* 2003, vol. 9, 583-588
- **Iruela-Arispe et al.** *Circulation,* 1999, vol. 100, 1423-1431
- **Lewis, TS. et al.** *Adv Canc Res,* 1998, vol. 74, 49-139
- **Hoshino, R. et al.** *Oncogene,* 1999, vol. 18, 813-822
- **Salh, B et al.** *Anticancer Res.,* 1999, vol. 19, 741-48
- **Sivaraman, VS et al.** *J. Clin. Invest.,* 1997, vol. 99, 1478-483
- **Mandell, JW et al.** *Am. J. Patrol.,* 1998, vol. 153, 1411-23
- **Licato, L.L. et al.** *Digestive Diseases and Sciences,* 1998, vol. 43, 1454-1464
- **Miyazawa et al.** *Biochem. Biophys. Res. Comm.,* 1989, vol. 163, 967-973

- **Nakamura et al.** *Nature,* 1989, vol. 342, 440-443
- **Seki et al.** *Biochem Biophys. Res. Commun.,* 1990, vol. 172, 321-327
- *Proc Natl Acad Sci USA,* 1991, vol. 88, 415-419
- *Science,* 1991, vol. 254, 1382-5
- **Rodrigues et al.** *Mol. Cell. Biol.,* 1991, vol. 11, 2962-2970
- **Park et al.** *Proc Natl Acad Sci USA,* 1987, vol. 84, 6379-6383
- **Ponzetto et al.** *Oncogene,* 1991, vol. 6, 553-559
- **Koo, H.-M. et al.** *Canc Res,* 1996, vol. 56, 5211-5216
- **Monks, A. et al.** *J Natl Canc Inst,* 1991, vol. 83, 757-766
- **Grever, M.R. et al.** *Sem Oncol,* 1992, vol. 19, 622-638
- **Duesbery, N.S. et al.** *Science,* 1998, vol. 280, 734-737
- **Vitale, G. et al.** *Biochem Biophys Res Comm,* 1998, vol. 248, 706-711
- **Duesbery, NS et al.** *CMLS Cell. Mol. Life Sci.,* 1999, vol. 55, 1599-1609
- **Duesbery et al.** *Proc. Natl. Acad. Sci. USA,* vol. 98, 4098-4094
- **Schulz, GE et al.** Pr-inciples of Protein Structure. Springer-Verlag, 1978
- **Creighton, T.E.** Proteins: Structure and Molecular Properties. W.H. Freeman & Co, 1983
- Anthrax Toxins. **Leppla, SH et al.** Handbook of Natural Toxins: Bacterial Toxins and Virulence Factors in Disease. Dekker, 1995
- **Singh, Y et al.** *J. Biol. Chem.,* 1989, vol. 264, 19103-19107
- **Novak, J. et al.** *J. Biol. Chem.,* 1992, vol. 267, 17186-17193
- **Gordon et al.** *Infect. Immun.,* 1988, vol. 56, 1066-1069
- **Milne et al.** *J. Biol Chem.,* 1994, vol. 269, 20607-20612
- Remington's Pharmaceutical Sciences. Mack Publishing Company, 1990
- **Sebolt-Leopold, JS et al.** *Nature Med.,* 1999, vol. 5, 810-816
- **Dudley, D.T. et al.** *Proc Nat'l Acad Sci USA,* 1995, vol. 92, 7686-7689
- **Alessi, D.R. et al.** *J Biol Chem,* 1995, vol. 270, 27489-27494
- **Favata, M et al.** *J Biol. Chem.,* 1998, vol. 273, 18623-18632
- **Cuenda, A et al.** *FEBS Lett.,* 1995, vol. 364, 229-233
- **Wong AK et al.** *Proc Natl Acad Sci USA,* 2001, 7481-7486
- **Wulff C et al.** *Endocrinology,* 2002, vol. 143, 2797-807
- **Holash, J et al.** *Proc Natl Acad Sci USA.,* 2002, vol. 99, 11393-11398
- **Huang J et al.** *Proc Natl Acad Sci USA,* 2003, vol. 100, 7785-90
- *Proc Natl Acad Sci USA,* 2003, vol. 100, 8624-5
- **Sharp, P.A.** *Genes Dev.,* 2001, vol. 15, 485-490
- **Bernstein, E et al.** *Nature,* 2001, vol. 409, 363-366
- **Nykanen, A et al.** *Cell,* 2001, vol. 107, 309-321
- **Elbashir, S.M. et al.** *Genes Dev.,* 2001, vol. 15, 188-200
- **Khvorova, A et al.** *Cell,* 2003, vol. 115, 209-216
- **Schwarz, DS et al.** *CELL,* 2003, vol. 115, 199-208
- **Far, RK et al.** *Nuc Acids Res,* 2003, 314417-4424
- **Reynolds, A et al.** *Nature Biotech.,* 2004, vol. 22, 326-330
- **Allshire.** *Science,* 2002, vol. 297, 1818-1819
- **Volpe et al.** *Science,* 2002, vol. 297, 1833-1837
- **Jenuwein.** *Science,* 2002, vol. 297, 2215-2218
- **Hall et al.** *Science,* 2002, vol. 297, 2232-2237
- **Martinez et al.** *Cell,* 2002, vol. 110, 563-574
- **Schwarz et al.** *Molecular Cell,* 2002, vol. 10, 537-568
- **Vickers et al.** *J Biol Chem,* 2003, vol. 278, 7108-7118
- **Yang et al.** *Proc Natl Acad Sci USA,* 2003, vol. 99, 9942-9947
- **Far et al.** *Nuc. Acids Res.,* 2003, vol. 31, 4417-4424
- **Reynolds et al.** *Nature Biotechnology,* 2004, vol. 22, 326-330
- **Tuschl et al.** *Genes & Dev.,* 1999, vol. 13, 3191-3197
- **Kawasaki et al.** *Nucleic Acids Res,* 2003, vol. 31, 700-707
- **Miyagishi et al.** *Nature Biotechnol,* 2003, vol. 20, 497-500
- **Lee et al.** *Nature Biotechnol,* 2002, vol. 20, 500-505
- **Brummelkamp et al.** *Science,* 2002, vol. 296, 550-553
- **McManus et al.** *RNA,* 2002, vol. 8, 842-850
- **Paddison et al.** *Gene Dev,* 2002, vol. 16, 948-958
- **Paddison et al.** *Proc Natl Acad Sci USA,* 2002, vol. 99, 1443-1448

- **Paul et al.** *Nature Biotechnol,* 2002, vol. 20, 505-508
- **Sui et al.** *Proc Natl Acad Sci USA,* 2002, vol. 99, 5515-5520
- **Yu et al.** *Proc Natl Acad Sci USA,* 2002, vol. 99, 6047-6052
- **Lau et al.** *Scientific American,* 2003, 34-41
- **McManus et al.** *Nature Reviews Genetics,* 2002, vol. 3, 737-747
- **Dykxhoorn et al.** *Nature Reviews Molecular Cell Biology,* 2003, vol. 4, 457-467
- **Hutvagner et al.** *Science,* 2002, vol. 297, 2056-60
- **Reinhart et al.** *Gene & Dev.,* 2002, vol. 16, 1616-1626
- **Reinhart et al.** *Science,* 2002, vol. 297, 1831
- **Fire et al.** *Nature,* 1998, vol. 391, 806-811
- **Moss.** *Curr Biol,* 2001, vol. 11, R772-5
- **Brummelkamp et al.** *Science,* 2002, vol. 296, 550-3
- **Bass.** *Nature,* 2001, vol. 411, 428-429
- **Elbashir et al.** *Nature,* 2001, vol. 411, 494-498
- **Gribskov ; Devereux.** Sequence Analysis Primer. Stockton Press, 1991
- **Cao, B et al.** *Proc. Natl. Acad. Sci.,* 2001, vol. 98, 7443-7448
- **Date, K. et al.** *Oncogene,* 1998, vol. 17, 3045-3054
- **Abounader, R et al.** *FASEB J.,* 2002, vol. 16, 108-110
- **Webb, CP et al.** *Cancer Res.,* 2000, vol. 60, 342-349
- **Atabey, N et al.** *J. Biol. Chem.,* 2001, vol. 276, 14308-14314
- **Christensen, JG et al.** *Cancer Res.,* 2003, vol. 63, 7345-7355
- **Prat et al.** *Mol Cell Biol,* 1991, vol. 11, 5954-5962
- **Prat et al.** *Int J Canc,* 1991, vol. 49, 323-328
- **Yamada et al.** *Brain Res,* 1994, vol. 637, 308-312
- **Crepaldi et al.** *J Cell Biol,* 1994, vol. 125, 313-320
- **Bottaro et al.** *Science,* 1991, vol. 251, 801-804
- **Silvagno et al.** *Arterioscler Thromb Vasc Biol,* 1995, vol. 15, 1857-1865
- **Kohler ; Milstein.** *Nature,* 1975, vol. 256, 495-497
- **Harlow, E. et al.** Antibodies: A Laboratory Manual. Cold Spring Harbor Laboratory Press, 1988
- Monoclonal Antibodies and Hybridomas: A New Dimension in Biological Analyses. Plenum Press, 1980
- **H. Zola et al.** Monoclonal Hybridoma Antibodies: Techniques and Applications. CRC Press, 1982
- **Cabilly et al.** *Proc. Natl. Acad. Sci. USA,* 1984, vol. 81, 3273-3277
- **Morrison et al.** *Proc. Natl. Acad. Sci. USA,* 1984, vol. 81, 6851-6855
- **Boulianne et al.** *Nature,* 1984, vol. 312, 643-646
- **Neuberger et al.** *Nature,* 1985, vol. 314, 268-270
- **Sahagan et al.** *J. Immunol.,* 1986, vol. 137, 1066-1074
- **Liu et al.** *Proc. Natl. Acad. Sci. USA,* 1987, vol. 84, 3439-3443
- **Better et al.** *Science,* 1988, vol. 240, 1041-1043
- **Jones et al.** *Nature,* 1986, vol. 321, 522-525
- **Reichmann et al.** *Nature,* 1988, vol. 332, 323-327
- **Presta.** *Curr. Op. Struct. Biol,* 1992, vol. 2, 593-596
- **Verhoeyen et al.** *Science,* 1988, vol. 239, 1534-1536
- **Sims et al.** *J. Immunol.,* 1993, vol. 151, 2296
- **Chothia et al.** *J. Mol. Biol.,* 1987, vol. 196, 901
- **Carter et al.** *Proc. Natl. Acad. Sci. USA,* 1992, vol. 89, 4285
- **Presta et al.** *J. Immunol.,* 1993, vol. 151, 2623-2632
- **Jakobovits et al.** *Proc. Natl. Acad. Sci. USA,* 1993, vol. 90, 2551-255
- **Jakobovits et al.** *Nature,* 1993, vol. 362, 255-258
- **Bruggermann et al.** *Year in Immunol.,* 1993, vol. 7, 33
- **Hoogenboom et al.** *J. Mol. Biol.,* 1991, vol. 222, 381
- **Marks et al.** *J. Mol. Bio.,* 1991, vol. 222, 581
- **Cole et al.** Monoclonal Antibodies and Cancer Therapy. Alan R. Liss, 1985, 77
- **Boerner et al.** *J. Immunol,* 1991, vol. 147, 86-95
- **Lu et al.** *FEBS Lett,* 1994, vol. 356, 56-59
- **Capon et al.** *Nature,* 1989, vol. 337, 525
- **Byrn et al.** *Nature,* 1990, vol. 344, 667
- **Hollinger et al.** *Proc. Natl. Acad. Sci,* 1993, vol. 90, 6444-6448
- **T. E. Creighton.** Proteins: Structure and Molecular Properties. W. H. Freeman & Co, 1983
- **Aplin et al.** *CRC Crit. Rev. Biochem.,* 1981, 259-306
- **Hakimuddin et al.** *Arch. Biochem. Biophys.,* 1987, vol. 259, 52
- **Edge et al.** *Anal. Biochem.,* 1981, vol. 118, 131
- **Thotakura et al.** *Meth. Enzymol.,* 1987, vol. 138, 350
- **Duskin et al.** *J Biol Chem,* 1982, vol. 257, 3105

- Protocols for Screening Chemical Agents and Natural Products Against Animal Tumors and Other Biological Systems. **Geran, R.I. et al.** Canc. Chemother. Reports. vol. 3, 1-112
- Human Tumor Xenograft Models in NCI Drug Development. **Plowman, J. et al.** Anticancer Drug Development Guide: Preclinical Screening, Clinical Trials and Approval, Part II: In Vivo Methods. Humana Press Inc, 1997
- Biometrik Tables for Statisticians. Cambridge Press, vol. 1, 165
- *J Natl Cancer Inst.,* 1953, vol. 13, 1328
- Handbook on Genetically Standardized Jax Mice. Jackson Memorial Laboratory, 1962
- *Ann NY Acad Sci,* 1963, vol. 100
- *Cancer Res,* 1955, vol. 15, 39
- **Malave, I. et al.** *J. Nat'l. Canc. Inst.,* 1979, vol. 62, 83-88
- **Gorelik, E. et al.** *J. Nat'l. Canc. Inst.,* 1980, vol. 65, 1257-1264
- **Gorelik, E. et al.** *Rec. Results Canc. Res.,* 1980, vol. 75, 20-28
- **Isakov, N. et al.** *Invasion Metas.,* 1982, vol. 2, 12-32
- **Talmadge J.E. et al.** *J. Nat'l. Canc. Inst.,* 1982, vol. 69, 975-980
- **Hilgard, P. et al.** *Br. J. Cancer,* 1977, vol. 35, 78-86
- **Thakur, M.L. et al.** *J. Lab. Clin. Med.,* 1977, vol. 89, 217-228
- **Grever, MR.** *Semin Oncol.,* 1992, vol. 19, 622-638
- **Venditti, J.M. et al.** Adv. Pharmacol and Chemother. 1984, vol. 2, 1-20
- **Rygaard, J. et al.** *Acta Pathol. Microbiol. Scand.,* 1969, vol. 77, 758-760
- **Giovanella, G.C. et al.** *J. Natl Canc. Inst.,* 1973, vol. 51, 615-619
- Cancer: Principles and Practice of Oncology, Updates. **Boyd, MR et al.** Anticancer Drug Development Guide: Preclinical Screening, Clinical Trials and Approval. Lippincott, 1989, vol. 3, 1-12
- **Stinson SF et al.** *Anticancer Res,* 1992, vol. 12, 1035-1054
- **Martin DS et al.** *Cancer Treat Rep,* 1984, vol. 68, 37-38
- **Martin DS et al.** *Cancer Res.,* 1986, vol. 46, 2189-2192
- **Stolfi, RL et al.** *J. Natl Canc Inst,* 1988, vol. 80, 52-55
- **Alley, MC et al.** *Canc Res,* 1991, vol. 51, 1247-1256
- **Crowley, C.W. et al.** *Proc. Natl. Acad. Sci. USA,* 1993, vol. 90, 5021-5025
- **Safrians, S. et al.** *Int'l J. Canc.,* 1996, vol. 66, 131-1f58
- **Fu, Y.S. et al.** *Anat. Quant. Cytol. Histol.,* 1989, vol. 11, 187-195
- **Hendrix, M.J.C. et al.** *Cancer Lett.,* 1987, vol. 38, 137-147
- **Albini, A. et al.** *Cancer Res.,* 1987, vol. 47, 3239-3245
- **Melchiori, A.** *Cancer Res.,* 1992, vol. 52, 2353-2356
- **Herron, G.S. et al.** *J. Biol. Chem.,* 1986, vol. 261, 2814-2818
- **Ballin, M. et al.** *Biochem. Biophys. Res. Comm.,* 1988, vol. 154, 832-838
- **L. Trusolino ; P.M. Comoglio.** *Nat Rev Cancer,* 2002, vol. 4, 289-300
- **G. F. Vande Woude et al.** CIBA Found. Symp.: Plasminogen-Related Growth Factors. Wiley, 1997, vol. 212, 119-132
- **D. Hanahan ; R. A. Weinberg.** *Cell,* 2000, vol. 100, 57-70
- **D. Hanahan ; J. Folkman.** *Cell,* 1996, vol. 86, 353-364
- **N. Ferrara.** *Semin. Oncol.,* 2002, vol. 29, 10-14
- **I. Sargiannidou ; J. Zhou ; G. P. Tuszynski.** *Exp. Biol. Med.,* 2001, vol. 226, 726-733
- **B. Jimenez et al.** *Nat. Med.,* 2000, vol. 6, 41-48
- **K. M. Dameron ; O. V. Volpert ; M. A. Tainsky ; N. Bouck.** *Science,* 1994, vol. 265, 1582-1584
- **K. Bleuel et al.** *Proc. Natl. Acad. Sci. U S A,* 1999, vol. 96, 2065-2070
- **F. Bussolino et al.** *J. Cell Biol.,* 1992, vol. 119, 629-641
- **D. S. Grant et al.** *Proc. Natl. Acad. Sci. U S A,* 1993, vol. 90, 1937-1941
- **E. M. Rosen ; I. D. Goldberg.** *Adv. Cancer. Res.,* 1995, vol. 67, 257-279
- **G. Dong et al.** *Cancer Res.,* 2001, vol. 61, 5911-5918
- **T. Moriyama et al.** *Biochem. Biophys. Res. Commun.,* 1998, vol. 249, 73-77
- **M. Jeffers ; S. Rong ; G. F. Vande Woude.** *Mol Cell Biol.,* 1996, vol. 16, 1115-1125
- **K. A. Furge ; Y. W. Zhang ; G. F. Vande Woude.** *Oncogene,* 2000, vol. 19, 5582-5589
- **Y. W. Zhang ; L. M. Wang ; R. Jove ; G. F. Vande Woude.** *Oncogene,* 2002, vol. 21, 217-226
- **G. Niu et al.** *Oncogene,* 2002, vol. 21, 2000-2008
- **J. Rak et al.** *Cancer Res.,* 2000, vol. 60, 490-498
- **T. A. Baudino et al.** *Genes Dev.,* 2002, vol. 16, 2530-2543
- **R. Kerbel ; J. Folkman.** *Nat. Rev. Cancer,* 2002, vol. 2, 727-739
- **N. Ferrara.** *Nat. Rev. Cancer,* 2002, vol. 2, 795-803
- **T. S. Lewis ; P. S. Shapiro ; N. G. Ahn.** *Adv. Cancer Res.,* 1998, vol. 74, 49-139

- **N. S. Duesbery et al.** *Science,* 1998, vol. 280, 734-737
- **N. S. Duesbery et al.** *Proc. Natl. Acad. Sci. U S A,* 2001, vol. 98, 4089-4094
- **B. Cao et al.** *Proc. Natl. Acad. Sci. U S A,* 2001, vol. 98, 7443-7448
- **K. Kuba et al.** *Cancer Res.,* 2000, vol. 60, 6737-6743